# FASCICULE DE BREVET EUROPEEN

(11) **EP 0 891 181 B1**
(45) Date de publication et mention de la délivrance du brevet: **14.05.2003**
(21) Numéro de dépôt: 97915539.7
(22) Date de dépôt: 21.03.1997
(51) Int. Cl.: A61K 7/13

(54) **COMPOSITIONS DE TEINTURE DES FIBRES KERATINIQUES CONTENANT DES PYRAZOLO-AZOLES; LEUR UTILISATION POUR LA TEINTURE COMME COUPLEURS, PROCEDE DE TEINTURE**
ZUSAMMENSETZUNG ZUR FÄRBUNG VON KERATINISCHEN FASERN ENTHALTEND PYRAZOLOAZOLE ALS KUPPLER, UND FÄRBEVERFAHREN
KERATIN FIBRE DYE COMPOSITION CONTAINING PYRAZOLO-AZOLE COMPOUNDS, USE THEREOF AS DYE COUPLERS, AND DYEING METHOD

(30) Priorité: 22.03.1996 FR 9603626
(43) Date de publication de la demande: 20.01.1999
(73) Titulaire: L'OREAL, 75008 Paris (FR)
(72) Inventeur: VIDAL, Laurent, F-75013 Paris (FR); MALLE, Gérard, F-77580 Villiers sur Morin (FR); MONTEIL, Eric, F-94300 Vincennes (FR)
(74) Mandataire: Miszputen, Laurent
(86) Numéro de dépôt international: FR9700507
(87) Numéro de publication internationale: WO97035551

(56) Documents cités:
- EP-A- 0 030 680
- WO-A-92/04883
- DE-A- 4 133 957

## Description

L'invention a pour objet une composition pour la teinture par oxydation des fibres kératiniques en particulier des cheveux humains contenant au moins un composé pyrazolo-azole comme coupleur et au moins une base d'oxydation.

Il est connu de teindre les fibres kératiniques et en particulier les cheveux humains avec des compositions tinctoriales contenant des précurseurs de colorant d'oxydation, en particulier des ortho ou paraphénylénediamines, des ortho ou paraaminophénols, des composés hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est-à-dire permettre d'obtenir des écarts de coloration les plus faibles possible tout au long d'une même fibre kératinique, qui peut être en effet, différemment sensibilisée (i.e. abîmée) entre sa pointe et sa racine.

Or, la demanderesse vient maintenant de découvrir qu'il est possible d'obtenir de nouvelles teintures puissantes, peu sélectives et particulièrement résistantes, capables d'engendrer des colorations intenses dans des nuances variées, en utilisant des composés pyrazolo-azoles comme coupleurs en présence d'une base d'oxydation.

Cette découverte est à la base de la présente invention.

L'invention a pour objet une composition pour la teinture des fibres kératiniques et en particulier des fibres kératiniques humaines telles que les cheveux, caractérisée par le fait qu'elle comprend, dans un milieu approprié pour la teinture :
- à titre de coupleur, au moins un composé pyrazolo-azole de formule (I) ou l'un de ses sels d'addition avec un acide : 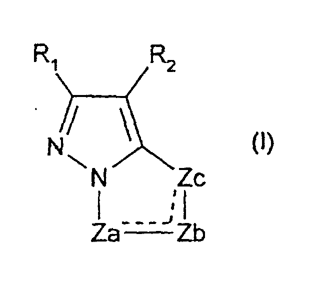 dans laquelle :
   . R₁ représente : un atome d'hydrogène ; un radical alkyle en C₁-C₂₀, linéaire ou ramifié, éventuellement substitué par 1 ou 2 radicaux R choisis dans le groupe constitué par halogène, nitro, cyano, hydroxy, alcoxy, aryloxy, amino, alkylamino, acylamino, carbamoyle, sulfonamido, sulfamoyle, imido, alkylthio, arylthio, aryle, alcoxycarbonyle, acyle ; un radical aryle (tel que phényle ou naphtyle), éventuellement substitué par 1 ou 2 radicaux R tels que précédemment définis ; un hétérocycle à 5 ou 6 chaînons possédant au moins un atome d'azote, d'oxygène ou de soufre (tel que pyridyle, quinolyle, pyrrolyle, morpholyle, furanyle, tétrahydrofuranyle, pyrazolyle, triazolyle, tétrazolyle, thiazolyle, oxazolyle, imidazolyle ou thiadiazolyle), éventuellement substitué par 1 ou 2 radicaux R tels que définis précédemment ;
      lorsque R₁ désigne un radical alkyle, un radical aryle ou un hétérocycle à 5 ou 6 chaînons (définis ci-dessus), il peut être relié à l'atome de carbone du noyau par l'intermédiaire d'un atome d'oxygène, d'azote ou de soufre (dans ce cas, R₁ devient XR₁ avec X = O, NH, S) ;
      R₁ peut désigner aussi un atome d'halogène (tel que brome, chlore ou fluor) ; un radical acyle ; un radical sulfonyle ; un radical sulfinyle ; un radical phosphonyle, un radical carbamoyle ; un radical sulfamoyle ; un radical cyano ; un radical siloxy, un radical amino ; un radical acylamino ; un radical acyloxy ; un radical carbamoyloxy ; un radical sulfonamide ; un radical imide ; un radical uréido ; un radical sulfamoylamino ; un radical alcoxycarbonylamino ; un radical aryloxycarbonylamino ; un radical alcoxycarbonyle ; un radical aryloxycarbonyle ; un radical carboxyle ;
   . R₂ représente : un atome d'hydrogène ; un atome d'halogène tel que brome, chlore ou fluor ; un groupe acétylamido ; un radical alcoxy (tel que par exemple : méthoxy, éthoxy, propyloxy, benzyloxy, méthoxyéthoxy, phénoxyéthoxy, 2-cyanoéthoxy, phénéthyloxy, p-chlorobenzyloxy, méthoxyéthylcarbamoylméthoxy) ; un radical aryloxy (tel que par exemple : phénoxy, 4-méthoxyphénoxy, 4-nitrophénoxy, 4-cyanophénoxy, 4-méthanesulfonamidophénoxy, 4-méthanesulfonylphénoxy, 3-méthylphénoxy, 1-naphtyloxy) ; un radical acyloxy (tel que par exemple : acétoxy, propanoyloxy, benzoyloxy, 2,4-dichlorobenzoyloxy, éthoxyalkyloxy, pyruviloyloxy, cinnamoyloxy, myristoyloxy) ; un radical arylthio (tel que par exemple : phénylthio, 4-carboxy-phénylthio, 2-éthoxy 5-tert-butylphénylthio, 2-carboxyphénylthio, 4-méthane-sulfonyl-phénylthio) ; un radical alkylthio (tel que par exemple : méthylthio, éthylthio, propylthio, butylthio, 2-cyanoéthylthio, benzylthio, phénéthylthio, 2-(diéthylamino) éthylthio, éthoxyéthylthio, phénoxyéthylthio) ; un radical hétéroarylthio (tel que par exemple : 5-phényl 2,3,4,5-tétrazolylthio, 2-benzothiazolylthio); un radical hétéroaryloxy (tel que par exemple : 5-phényl 2,3,4,5-tétrazolyloxy, 2-benzo-thiazolyloxy) ; un radical thiocyano ; un radical N,N-diéthyl thiocarbonylthio ; un radical dodécyl-oxythio carbonylthio ; un radical benzènesulfonamido ; un radical N-éthyltoluène sulfonamido ; un radical pentafluorobutanamido ; un radical 2,3,4,5,6-pentafluorobenzamido ; un radical p-cyanophényluréido, un radical N,N-diéthyl-sulfamoylamino ; un radical pyrazolyle ; un radical imidazolyle ; un radical triazolyle ; un radical tétrazolyle ; un radical benzimidazolyle ; un radical 1-benzyl 5-éthoxy 3-hydantoïnyte ; un radical 1-benzyl 3-hydantoïnyle ; 5,5-diméthyl 2,4-dioxo 3-oxazolidinyle ; un radical 2-oxy 1,2-dihydro 1-pyridinyle ; un alkylamido ; un arylamido ; un radical NR^{III}R^{IV} avec R^{III} et R^{IV} représentant, identiques ou différents, un alkyle en C₁-C₄ ; un hydroxyalkyle ; un carboxyle ; ou un radical alcoxycarboxylique.
      Zₐ, Z_{b}, Z_{c} représentent, indépendamment l'un de l'autre, un atome d'azote, un atome de carbone portant un radical R₃ ou R₄ ayant les mêmes significations que celles indiquées pour le radical R₁ ; sous réserve que l'un au moins des radicaux Zₐ, Z_{b} et Z_{c} soit différent d'un atome de carbone ; R₃ et R₄ peuvent également former entre eux un cycle aromatique substitué ou non tel que phényle ;
   . et au moins une base d'oxydation.

Les sels d'addition avec un acide des composés de l'invention peuvent être choisis notamment parmi des chlorhydrates, les bromhydrates, les tartrates, les tosylates, les benzènesulfonates, les sulfates, les lactates et les acétates.

Parmi les radicaux R₁ de la formule (I) définie ci-dessus, on préfère les radicaux choisis dans le groupe constitué par :
un atome d'hydrogène ; un alkyle en C₁-C₄, linéaire ou ramifié ; un phényle ; un phényle substitué par un atome d'halogène, un alkyle en C₁-C₄, un alcoxy en C₁-C₄, un groupe nitro, un groupe amino, un groupe trifluorométhyle ou alkylamino en C₁-C₄ ; un radical benzyle ; un radical benzyle substitué par un atome d'halogène, un alkyle en C₁-C₄, un alcoxy en C₁-C₄, un groupe nitro, un groupe amino, un groupe trifluorométhyle ; un alkylamino en C₁-C₄ ; un hétérocycle choisi parmi le thiophène, le furane ou la pyridine ; un radical trifluorométhyle ; un radical (CH₂)ₚ-X-(CH₂)_{q}-OR' où p et q sont entiers, identiques ou différents, compris entre 1 et 3, R' représente H ou méthyle et X désigne un atome d'oxygène ou un groupe NR" avec R" désignant hydrogène ou méthyle ; un hydroxyalkyle en C₁-C₄ ; un aminoalkyle en C₁-C₄ ; un alkylamino en C₁-C₄ ; un dialkylamino en C₁-C₄ ; un arylamino ; un radical alcoxy choisi parmi méthoxy, éthoxy et phénoxy ; un halogène choisi parmi fluor, chlore et brome ; un groupe carboxyle ; un alcoxycarbonyle en C₁-C₄ ; un phényloxycarbonyle ; méthylthio ; éthylthio ; phénylthio ; méthanesulfonyle ; cyano.

Parmi les radicaux R₁ de la formule (I) définie ci-dessus, on préfère plus particulièrement les radicaux choisis dans le groupe constitué par :
hydrogène ; un alkyle choisi parmi méthyle, éthyle, isopropyle, ter-butyle ; un halogène choisi parmi fluor et chlore ; phényle ; toluyle ; 4-chlorophényle ; 4-méthoxyphényle ; 3-méthoxyphényle ; 2-méthoxyphényle ; benzyle ; un hétérocycle choisi parmi pyridyle, furyle ou thiènyle ; trifluorométhyle ; hydroxyméthyle ; aminométhyle ; méthoxy ou éthoxy ; méthylamino ou éthylamino ou diméthylamino ; carboxyle ; méthoxycarbonyle ou éthoxycarbonyle ; cyano.

Et encore plus particulièrement , on préfère les radicaux R₁ choisis dans le groupe constitué par : hydrogène ; méthyle ; éthyle ; phényle ; toluyle ; 4-chlorophényle ;4-méthoxyphényle ; benzyle ; trifluorométhyle ; chloro ; un radical méthoxy ou éthoxy ; un radical carboxyle ; méthylamino ou diméthylamino ; cyano.

Parmi les radicaux R₂ de la formule (I) définie ci-dessus, on préfère les radicaux choisis dans le groupe constitué par :
un atome d'hydrogène ; un alcoxy en C₁-C₄ ; phénoxy ; phénoxy substitué par un atome d'halogène, un alkyle en C₁-C₄, un carboxyle, un groupe trifluorométhyle ; un radical acyloxy ; benzyloxy ; alkylthio en C₁-C₄ ; phénylthio ; phénylthio substitué par un atome d'halogène, un alkyle en C₁-C₄, un carboxyle, un groupe trifluorométhyle ; un alkylamido en C₁-C₄ ; phénylamido ; un radical NR^{III}R^{IV} avec R^{III} et R^{IV} représentant, identiques ou différents, un alkyle en C₁-C₄ , un hydroxyalkyle en C₁-C₄ ; un carboxyle ; un radical alcoxycarboxylique en C₁-C₄.

Parmi les radicaux R₂ de la formule (I) définie ci-dessus, on préfère plus particulièrement les radicaux choisis dans le groupe constitué par :
hydrogène ; chlore ou brome ; méthoxy ou éthoxy ; phényloxy ; 4-méthylphényloxy ; acyloxy ; benzyloxy ; méthylthio ou éthylthio ; phénylthio ; 4-méthylphénylthio ; 2-tertio-butylphénylthio ; acétamido ; phénylacétamido ; diméthylamino ; diéthylamino ; éthyl-méthylamino ; (β-hydroxyéthyl)-méthylamino.

Et encore plus particulièrement , on préfère les radicaux R₂ choisis dans le groupe constitué par : hydrogène ; chlore ; éthoxy ; phénoxy ; benzyloxy ; acyloxy ; acétamido ; diméthylamino.

Parmi les radicaux R₃ et R₄ de la formule (I) définie ci-dessus, on préfère les radicaux choisis dans le groupe constitué par :
un atome d'hydrogène ; un alkyle en C₁-C₄, linéaire ou ramifié ; un radical trifluorométhyle ; un phényle ; un phényle substitué par un ou deux groupes choisis parmi un halogène, un alkyle en C₁-C₄, un alcoxy en C₁-C₄, un hydroxy, un carboxyle, un groupe nitro, un alkylthio en C₁-C₄, un groupe méthylènedioxy, un groupe amino, un groupe trifluorométhyle ou un alkylamino en C₁-C₄ ; un radical benzyle ; un radical benzyle substitué par un atome d'halogène, un méthyle ou isopropyle, méthoxy ; un hydroxyalkyle en C₁-C₄ ; un aminoalkyle en C₁-C₄ ; un alkylaminoalkyle en C₁-C₄ ; un radical alcoxy choisi parmi méthoxy, éthoxy et phénoxy ; méthylthio ; éthylthio ; phénylthio ; méthanesulfonyle ; un cycle aromatique substitué ou non formé par R₃ et R₄ tel que phényle, phényle substitué par un radical sulfonyle, un halogène, un alcoxy en C₁-C₄, un alkyle en C₁-C₄, nitro, cyano, amino, alkylamino, trifluorométhyle.

Parmi les radicaux R₃ et R₄ de la formule (I) définie ci-dessus, on préfère plus particulièrement les radicaux choisis dans le groupe constitué par : hydrogène ; un alkyle choisi parmi méthyle, éthyle, isopropyle, n-propyle, ter-butyle ; phényle ; toluyle ; 2-, 3- ou 4-chlorophényle ; 3- ou 4-hydroxyphényle ; 3- ou 4-aminophényle ; 3- ou 4-méthoxyphényle ; 4-trifluorométhylphényle ; benzyle ; trifluorométhyle ; hydroxyméthyle ; hydroxyéthyle ; hydroxyisopropyle ; aminométhyle ou aminoéthyle ; méthoxy ou éthoxy ; méthylthio ou éthylthio ; un cycle aromatique substitué ou non formé par R₃ et R₄ tel que phényle, toluyle, sulfonylphényle, chlorophényle.

Et encore plus particulièrement , on préfère les radicaux R₃ et R₄ choisis dans le groupe constitué par : hydrogène ; méthyle ; éthyle ; isopropyle ; phényle ; 4-chlorophényle ; 4-méthoxyphényle ; 4-aminophényle ; méthoxy ou éthoxy ; méthylthio ou éthylthio ; un cycle phényle formé par R₃ et R₄.

Parmi les composés de l'invention de formule (I) préférentiels, on peut citer ceux choisis dans le groupe constitué par :
i) les pyrazolo-[1,5-b] 1,2,4-triazoles de formule : 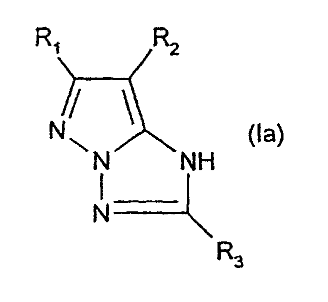
ii) les pyrazolo [3,2-c] 1,2,4-triazoles de formule : 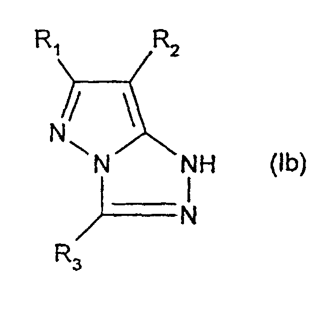
iii) les pyrazolotétrazoles de formule : 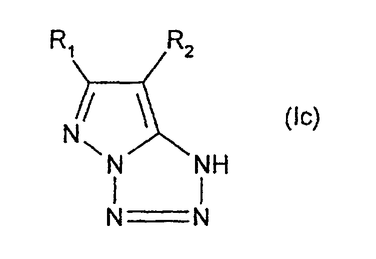
iv) les pyrazolo-[1,5-a]-imidazoles de formule : 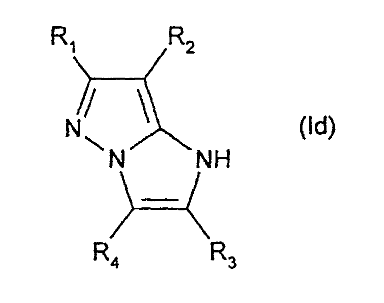
v) les pyrazolo-[5,1-e]-pyrazoles de formule : 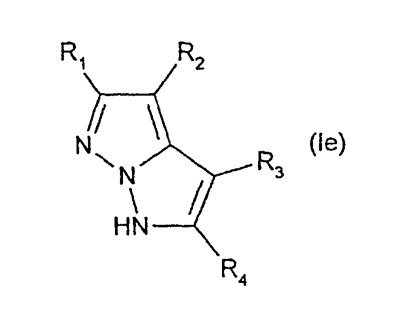
vi) les pyrazolo-[5, 1-e]-1,2,3-triazoles de formule : 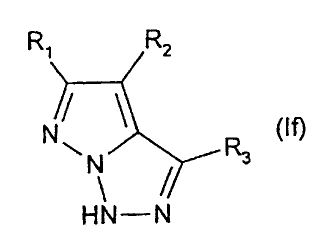
dans lesquelles R₁, R₂, R₃ et R₄ ont les mêmes significations indiquées ci-dessus dans la formule (I).

Comme exemples de composés de formule (la) ou (lb), on peut citer en particulier ceux pour lesquels :
- R₁ désigne hydrogène, méthyle, éthylthio, amino, trifluorométhyle, carboxyle, phényle, éthoxy ou cyano
- R₂ désigne hydrogène ou chlore ;
- R₃ hydrogène, méthyle, éthyle, isopropyle, β-aminoéthyle, β-hydroxyéthyle, phényle, méthylthio ou éthoxy.

Parmi les composés de formule (la) ci-dessus, on peut tout particulièrement citer :
- le 2-méthyl pyrazolo [1,5-b]-1,2,4-triazole,
- le 2-éthyl pyrazolo [1,5-b]-1,2,4-triazole,
- le 2-isopropyl pyrazolo [1,5-b]-1,2,4-triazole,
- le 2-phényl pyrazolo [1,5-b]-1,2,4-triazole,
- le 2,6-diméthyl pyrazolo [1,5-b]-1,2,4-triazole ,
- le 6-méthyl-2-éthyl pyrazolo [1,5-b]-1,2,4-triazole,
- le 6-méthyl-2-isopropyl pyrazolo [1,5-b]-1,2,4-triazole,
- le 6-méthyl-2-phényl pyrazolo [1,5-b]-1,2,4-triazole,
- le 6-carboxy-2-méthyl pyrazolo [1,5-b]-1,2,4-triazole,
- le 6-carboxy-2-éthyl pyrazoio [1,5-b]-1,2,4-triazole,
- le 6-carboxy-2-isopropyl pyrazolo [1,5-b]-1,2,4-triazole,
- le 6-carboxy-2-phényl pyrazolo [1,5-b]-1,2,4-triazole,
- le 6-phényl-2-méthyl pyrazolo [1,5-b]-1,2,4-triazole,
- le 6-phényl-2-éthyl pyrazolo [1,5-b]-1,2,4-triazole,
- le 6-phényl-2-isopropyl pyrazolo [1,5-b]-1,2,4-triazole,
- le 6-phényl-2-phényl pyrazolo [1,5-b]-1,2,4-triazole,
- le 6-amino-2-méthyl pyrazolo [1,5-b]-1,2,4-triazole,
- le 6-amino-2-éthyl pyrazolo [1,5-b]-1,2,4-triazole,
- le 6-amino-2-isopropyl pyrazolo [1,5-b]-1,2,4-triazole,
- le 6-amino-2-phényl pyrazolo [1,5-b]-1,2,4-triazole,
- le 6-éthylthio-2-méthyl pyrazolo [1,5-b]-1,2,4-triazole,
- le 6-éthylthio-2-éthyl pyrazolo [1,5-b]-1,2,4-triazole,
- le 6-éthylthio-2-isopropyl pyrazolo [1,5-b]-1,2,4-triazole,
- le 6-éthylthio-2-phényl pyrazolo [1,5-b]-1,2,4-triazole,
- le 6-éthoxy-2-méthyl pyrazolo [1,5-b]-1,2,4-triazole,
- le 6-éthoxy-2-éthyl pyrazolo [1,5-b]-1,2,4-triazole,
- le 6-éthoxy-2-isopropopyl pyrazolo [1,5-b]-1,2,4-triazole,
- le 6-éthoxy-2-phényl pyrazolo [1,5-b]-1,2,4-triazole,
- le 6-méthyl-2-(2'-aminoéthyl) pyrazolo [1,5-b]-1,2,4-triazole,
- le 6-carboxy-2-(2'-aminoéthyl) pyrazolo [1,5-b]-1,2,4-triazole,
- le 6-phényl-2-(2'-aminoéthyl) pyrazolo [1,5-b]-1,2,4-triazole,
- le 6-éthylthio-2-(2'-aminoéthyl) pyrazolo [1,5-b]-1,2,4-triazole,
- le 2-(2'-aminoéthyl) pyrazolo [1,5-b]-1,2,4-triazole,
- le 2-(2'-hydroxyéthyl) pyrazolo [1,5-b]-1,2,4-triazole,
- le 6-méthyl-2-(2'-hydroxyéthyl) pyrazolo [1,5-b]-1,2,4-triazole,
- le 6-éthylthio-2-(2'-hydroxyéthyl) pyrazolo [1,5-b]-1,2,4-triazole,
- le 6-carboxy-2-(2'-hydroxyéthyl) pyrazolo [1,5-b]-1,2,4-triazole,
- le 6-phényl-2-(2'-hydroxyéthyl) pyrazolo [1,5-b]-1,2,4-triazole,
- le 7-chloro-2,6-diméthylpyrazolo[1,5-b]-1,2,4-triazole,
- le 7-bromo -2,6-diméthylpyrazolo[1,5-b]-1,2,4-triazole,
et leurs sels d'addition avec un acide.

Parmi les composés de formule (Ib) ci-dessus, on peut tout particulièrement citer :
- le 3-méthyl pyrazolo [3,2-c]-1,2,4-triazole,
- le 3-méthylsulfinyl-6-phényl-pyrazolo [3,2-c]-1,2,4-triazole,
- le 3-éthyl pyrazolo [3,2-c]-1,2,4-triazole,
- le 3-isopropyl pyrazolo [3,2-c]-1,2,4-triazole,
- le 3-phényl pyrazolo [3,2-c]-1,2,4-triazole,
- le 3-(2'-aminoéthyl) pyrazolo [3,2-c]-1,2,4-triazole,
- le 3-(2'-hydroxyéthyl) pyrazolo [3,2-c]-1,2,4-triazole,
- le 6-méthyl-3-éthyl pyrazolo [3,2-c]-1,2,4-triazole,
- le 3,6-diméthyl- pyrazolo [3,2-c]-1,2,4-triazole,
- le 6-méthyl-3-isopropyl- pyrazolo [3,2-c]-1,2,4-triazole,
- le 6-méthyl-3-phényl-pyrazolo [3,2-c]-1,2,4-triazole,
- le 6-méthyl-3-(2'-aminoéthyl)- pyrazolo [3,2-c]-1,2,4-triazole,
- le 6-méthyl-3-(2'hydroxyéthyl)- pyrazolo [3,2-c]-1,2,4-triazole,
- le 6-méthyl-3-méthylthio- pyrazolo [3,2-c]-1,2,4-triazole,
- le 6-phényl-3-méthyl- pyrazolo [3,2-c]-1,2,4-triazole,
- le 6-phényl-3-éthyl- pyrazolo [3,2-c]-1,2,4-triazole,
- le 6-phényl-3-isopropyl- pyrazolo [3,2-c]-1,2,4-triazole,
- le 6-phényl-3-phényl- pyrazolo [3,2-c]-1,2,4-triazole,
- le 6-phényl-3-(2'-aminoéthyl)-pyrazolo [3,2-c]-1,2,4-triazole,
- le 6-phényl-3-(2'-hydroxyéthyl)- pyrazolo [3,2-c]-1,2,4-triazole,
- le 6-phényl-3-méthylthio- pyrazolo [3,2-c]-1,2,4-triazole,
- le 6-éthylthio-3-méthyl- pyrazolo [3,2-c]-1,2,4-triazole,
- le 6-éthylthio-3-éthyl- pyrazolo [3,2-c]-1,2,4-triazole,
- le 6-éthylthio-3-isopropyl- pyrazolo [3,2-c]-1,2,4-triazole,
- le 6-éthylthio-3-phényl- pyrazolo [3,2-c]-1,2,4-triazote,
- le 6-éthylthio-3-(2'-aminoéthyl)- pyrazolo [3,2-c]-1,2,4-triazole,
- le 6-éthylthio-3-(2'-hydroxyéthyl)- pyrazolo [3,2-c]-1,2,4-triazole,
- le 6-trifluorométhyl-3-méthylthio-pyrazolo [3,2-c]-1,2,4-triazole,
- le 6-trifluorométhyl- pyrazolo [3,2-c]-1,2,4-triazole,
- le 6-carboxy-3-méthyl- pyrazolo [3,2-c]-1,2,4-triazole,
- le 6-carboxy-3-éthyl- pyrazolo [3,2-c]-1,2,4-triazole,
- le 6-carboxy-3-isopropyl- pyrazolo [3,2-c]-1,2,4-triazole,
- le 6-carboxy-3-phényl- pyrazolo [3,2-c]-1,2,4-triazole,
- le 6-carboxy-3-(2'amino-éthyl)- pyrazolo [3,2-c]-1,2,4-triazole,
- le 6-carboxy-3-(2'-hydroxyéthyl)- pyrazolo [3,2-c]-1,2,4-triazole,
- le 7-chloro-3,6-diméthylpyrazolo[3,2-c]-1,2,4-triazole,
- le 7-méthoxycarbonyl-3,6-diméthylpyrazolo [3,2-c]-1,2,4-triazole,
et leurs sels d'addition avec un acide.

Comme exemples de composés de formule (Ic), on peut citer en particulier ceux pour lesquels :
- R₁ désigne hydrogène, méthyle, trifluorométhyle, carboxyle, phényle, éthoxy ou cyano
- R₂ désigne hydrogène ou chlore ;

Parmi les composés de formule (lc) ci-dessus, on peut tout particulièrement citer :
- le pyrazolo [5,1-e]-tétrazole,
- le 6-méthyl pyrazolo [5,1-e]-tétrazole,
- le 6-phényl pyrazolo [5,1-e]-tétrazole,
- le 6-carboxy pyrazolo [5,1-e]-tétrazole,
- le 7-chloro-6-méthyl pyrazolo [5,1-e]-tétrazole,
et leurs sels d'addition avec un acide

Comme exemples de composés de formule (Id), on peut citer en particulier ceux pour lesquels :
- R₁ désigne hydrogène, méthyle, trifluorométhyle, amino, carboxyle, phényle, éthoxy ou cyano ;
- R₂ désigne hydrogène ou chlore ;
- R₃ et R₄ désignent respectivement hydrogène et hydrogène, hydrogène et méthyle, méthyle et hydrogène, hydrogène et amino, hydrogène et phényle ; R₃ et R₄ forment entre eux un cycle phényle.

Parmi les composés de formule (Id) ci-dessus, on peut tout particulièrement citer :
- le pyrazolo [1,5-a] imidazole,
- le 2-méthyl- pyrazolo [1,5-a] imidazole,
- le 2-phényl - pyrazolo [1,5-a] imidazole,
- le pyrazolo [1,5-a] benzimidazole,
- le 6-méthyl- pyrazolo [1,5-a] imidazole,
- le 2,6-diméthyl- pyrazolo [1,5-a] imidazole,
- le 6-méthyl-2-phényl- pyrazolo [1,5-a] imidazole,
- le 6-méthyl- pyrazolo [1,5-a] benzimidazole,
- le 6-phényl- pyrazolo [1,5-a] imidazole,
- le 6-phényl-2-méthyl- pyrazolo [1,5-a] imidazole,
- le 2,6-diphényl- pyrazolo [1,5-a] imidazole,
- le 6-phényl- pyrazolo [1,5-a] benzimidazole,
- le 6-carboxy- pyrazolo [1,5-a] imidazole,
- le 6-carboxy-2-méthyl- pyrazolo [1,5-a] imidazole,
- le 6-carboxy-2-phényl- pyrazolo [1,5-a] imidazole,
- le 6-carboxy- pyrazolo [1,5-a] benzimidazole,
- le 6-éthoxy- pyrazolo [1,5-a] imidazole,
- le 6-éthoxy-2-méthyl- pyrazolo [1,5-a] imidazole,
- le 6-éthoxy-2-phényl- pyrazolo [1,5-a] imidazole,
- le 6-trifluorométhyl- pyrazolo [1,5-a] benzimidazole,
- le 6-amino- pyrazolo [1,5-a] imidazole,
- le 6-amino-2-méthyl- pyrazolo [1,5-a] imidazole,
- le 6-amino-2-phényl - pyrazolo [1,5-a] imidazole,
- le 6-amino- pyrazolo [1,5-a] benzimidazole,
- le 6-éthylthio- pyrazolo [1,5-a] imidazole,
- le 6-éthylthio-2-méthyl- pyrazolo [1,5-a] imidazole,
- le 6-éthylthio-2-phényl- pyrazolo [1,5-a] imidazole,
- le 7-chloro -6-méthyl- pyrazolo [1,5-a] imidazole,
- le 7-chloro -6-méthyl- pyrazolo [1,5-a] benzimidazole,
et leurs sels d'addition avec un acide.

Comme exemples de composés de formule (le), on peut citer en particulier ceux pour lesquels :
- R₁ désigne hydrogène, méthyle, trifluorométhyle, carboxyle, phényle, éthoxy ou cyano ;
- R₂ désigne hydrogène ou chlore ;
- R₃ et R₄ désignent respectivement hydrogène et méthyle, méthyle et hydrogène, méthyle et méthyle, hydrogène et phényle.

Parmi les composés de formule (le) ci-dessus, on peut tout particulièrement citer :
- le 8-amino-4-méthyl-pyrazolo [5,1-e]- pyrazole,
- le 8-amino-5-chloro-4-méthyl-pyrazolo [5,1-e]- pyrazole,
et leurs sels d'addition avec un acide.

Comme exemples de composés de formule (If), on peut citer en particulier ceux pour lesquels :
- R₁ désigne hydrogène, méthyle, trifluorométhyle, carboxyle, phényle, éthoxy ou cyano ;
- R₂ désigne hydrogène ou chlore ;
- R₃ hydrogène ou méthyle.

Parmi les composés de formule (If) ci-dessus, on peut tout particulièrement citer :
- le 5-méthylpyrazolo [5,1-e]-1, 2, 3 triazole,
- le 5-méthyl-6-chloro- pyrazolo [5,1-e]-1, 2, 3 triazole,
- le 5-phénylpyrazolo [5,1-e]-1, 2, 3 triazole,
et leurs sels d'addition avec un acide.

Un composé de formule (I) tout particulièrement préféré est celui de formule (la) suivant :

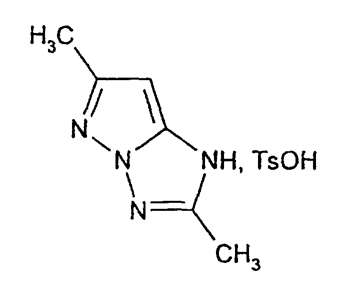

Les composés de la présente invention, leurs intermédiaires de synthèse et leurs procédés de préparation sont décrits dans les demandes de brevets et brevets suivants : FR 2 075 583, EP-A-119 860, EP-A-285 274, EP-A-244 160, EP-A-578 248, GB 1 458 377, US 3 227 554, US 3 419 391, US 3 061 432, US 4 500 630, US 3 725 067, US 3 926 631, US 5 457 210, JP 84/99437, JP 83/42045, JP 84/162548, JP 84/171956, JP 85/33552, JP 85/43659, JP 85/172982, JP 85/190779, ainsi que dans les publications suivantes : Chem. Ber. 32, 797 (1899), Chem. Ber. 89, 2550, (1956), J. Chem. Soc. Perkin trans I, 2047, (1977), J. Prakt. Chem., 320, 533, (1978), J. fur Chem., 326(5), 829, (1984).

Le ou les composés de formule (I) représentent de préférence de 0,0005 à 12 % en poids environ du poids total de la composition tinctoriale, et encore plus préférentiellement de 0,005 à 6 % en poids environ de ce poids.

La nature de la ou des bases d'oxydation pouvant être utilisées dans la composition tinctoriale selon l'invention n'est pas critique. Cette ou ces bases d'oxydation sont de préférence choisies parmi les paraphénylènediamines, les bis-phénylalkylènediamines, les paraaminophénols, les ortho-aminophénols, les bases hétérocycliques, et leurs sels d'addition avec un acide.

Parmi les paraphénylénediamines utilisables à titre de bases d'oxydation dans la composition tinctoriale selon l'invention, on peut notamment citer les composés répondant à la formule (II) suivante, et leurs sels d'addition avec un acide : 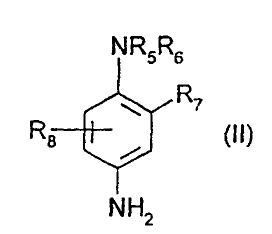 dans laquelle :
R₅ représente un atome d'hydrogène, un radical alkyle en C₁-C₄, monohydroxyalkyle en C₁-C₄, polyhydroxyalkyle en C₂-C₄ ou alcoxy(C₁-C₄)alkyle(C₁-C₄),
R₆ représente un atome d'hydrogène, un radical alkyle en C₁-C₄, monohydroxyalkyle en C₁-C₄ ou polyhydroxyalkyle en C₂-C₄,
R₇ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄,
R₈ représente un atome d'hydrogène ou un radical alkyle en C₁-C₄.

Dans la formule (II) ci-dessus, et lorsque R₇ est différent d'un atome d'hydrogène, alors R₅ et R₆ représentent de préférence un atome d'hydrogène et R₇ est de préférence identique à R₈, et lorsque R₇ représente un atome d'halogène, alors R₅, R₆ et R₈ représentent de préférence un atome d'hydrogène.

Parmi les paraphénylènediamines de formule (II) ci-dessus, on peut plus particulièrement citer la paraphénylènediamine, la paratoluylènediamine, la 2-isopropyl paraphénylène-diamine, la 2-β-hydroxyéthyl paraphénylènediamine, la 2-β-hydroxyéthyloxy para-phénylènediamine, la 2,6-diméthyl paraphénylènediamine, la 2,6-diéthyl paraphénylène-diamine, la 2,3-diméthyl paraphénylènediamine, la N,N-bis-(β-hydroxyéthyl) paraphénylènediamine, le 4-amino 1-(β-méthoxyéthyl)amino benzène, la 2-chloro para-phénylènediamine, et leurs sels d'addition avec un acide.

Parmi les bis-phénylalkylènediamines utilisables à titre de bases d'oxydation dans la composition tinctoriale selon l'invention, on peut notamment citer les composés répondant à la formule (III) suivante, et leurs sels d'addition avec un acide : 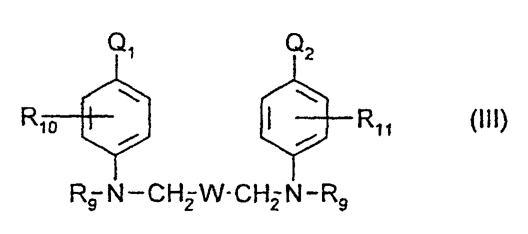 dans laquelle :
Q₁ et Q₂, identiques ou différents, représentent un radical hydroxyle ou NHR₁₂ dans lequel R₁₂ représente un atome d'hydrogène ou un radical alkyle en C₁-C₄,
R₉ représente un atome d'hydrogène, un radical alkyle en C₁-C₄, monohydroxyalkyle en C₁-C₄, polyhydroxyalkyle en C₂-C₄ ou aminoalkyle en C₁-C₄ dont le reste amino peut être substitué,
R₁₀ et R₁₁, identiques ou différents, représentent un atome d'hydrogène ou d'halogène ou un radical alkyle en C₁-C₄,
W représente un radical pris dans le groupe constitué par les radicaux suivants : 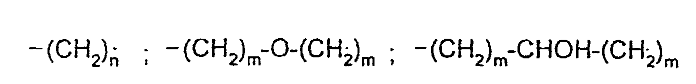 et 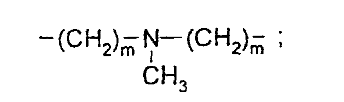
dans lesquels n est un nombre entier compris entre 0 et 8 inclusivement et m est un nombre entier compris entre 0 et 4 inclusivement.

Parmi les bis-phénylalkylènediamines de formule (III) ci-dessus, on peut plus particulièrement citer le N,N'-bis-(β-hydroxyéthyl) N,N'-bis-(4'-aminophényl) 1,3-diamino 2-propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi ces bis-phénylalkylènediamines de formule (III), le N,N'-bis-(β-hydroxyéthyl) N,N'-bis-(4'-aminophényl) 1,3-diamino propanol ou l'un de ses sels d'addition avec un acide sont particulièrement préférés.

Parmi les paraaminophénols utilisables à titre de bases d'oxydation dans la composition tinctoriale selon l'invention, on peut notamment citer les composés répondant à la formule (IV) suivante, et leurs sels d'addition avec un acide : 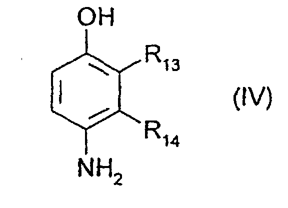 dans laquelle :
R₁₃ représente un atome d'hydrogène, un radical alkyle en C₁-C₄, monohydroxyalkyle en C₁-C₄, alcoxy(C₁-C₄)alkyle(C₁-C₄) ou aminoalkyle en C₁-C₄,
R₁₄ représente un atome d'hydrogène ou de fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₃ ou R₁₄ représente un atome d'hydrogène.

Parmi les paraaminophénols de formule (IV) ci-dessus, on peut plus particulièrement citer le paraaminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

Parmi les ortho-aminophénols utilisables à titre de bases d'oxydation dans la composition tinctoriale selon l'invention, on peut notamment citer le 2-amino phénol, le 2-amino 1-hydroxy 5-méthyl benzène, le 2-amino 1-hydroxy 6-méthyl benzène, le 5-acétamido 2-amino phénol, et leurs sels d'addition avec un acide.

Parmi les bases hétérocycliques utilisables à titre de bases d'oxydation dans la composition tinctoriale selon l'invention, on peut notamment citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diaminopyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-333 495, comme la 2,4,5,6-tétra-aminopyrimidine, la 4-hydroxy 2,5,6-triamino-pyrimidine, et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969 et WO 94/08970 comme le 4,5-diamino 1-méthyl pyrazole, le 3,4-diamino pyrazole et le 1-(4'-chlorobenzyl)-4,5-diaminopyrazole, et leurs sels d'addition avec un acide.

Selon l'invention, la ou les bases d'oxydation représentent de préférence de 0,0005 à 12 % en poids environ du poids total de la composition tinctoriale, et encore plus préférentiellement de 0,005 à 6 % en poids environ de ce poids.

La composition tinctoriale selon l'invention peut également renfermer un ou plusieurs coupleurs additionnels différents des composés de formule (I) et/ou un ou plusieurs colorants directs de façon à faire varier ou enrichir en reflets les nuances obtenues avec les bases d'oxydation.

Les coupleurs additionnels utilisables dans la composition selon l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, et leurs sels d'addition avec un acide.

Ces coupleurs peuvent notamment être choisis parmi le 2-méthyl 5-amino phénol, le 5-N-(β-hydroxyéthyl)amino 2-méthyl phénol, le 3-amino phénol, le 1,3-dihydroxybenzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, et leurs sels d'addition avec un acide.

Lorsqu'ils sont présents ces coupleurs additionnels représentent de préférence de 0,0005 à 5 % en poids environ du poids total de la composition tinctoriale, et encore plus préférentiellement de 0,005 à 3 % en poids environ de ce poids.

Les sels d'addition avec un acide de la ou des bases d'oxydation et/ou des coupleurs additionnels utilisables dans la composition tinctoriale de l'invention sont notamment choisis parmi les chlorhydrates, les bromhydrates, les sulfates et les tartrates, les lactates et les acétates.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcools inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (V) suivante : 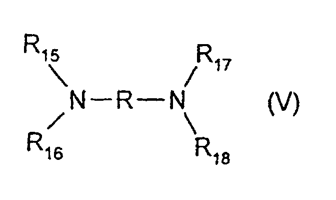 dans laquelle R est un reste propylène éventuellement substitué par un groupement hydroxyle ou un radical alkyle en C₁-C₄ ; R₁₅, R₁₆, R₁₇ et R₁₈, identiques ou différents, représentent un atome d'hydrogène, un radical alkyle en C₁-C₄ ou hydroxyalkyle en C₁-C₄.

La composition tinctoriale selon l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet l'utilisation des pyrazolo-azoles de formule (I) ci-dessus, à titre de coupleur, en association avec au moins une base d'oxydation pour la teinture d'oxydation des fibres kératiniques et particulier des fibres kératiniques humaines telles que les cheveux.

Un autre objet de l'invention est un procédé de teinture d'oxydation des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus.

Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent servent à illustrer l'invention sans toutefois présenter un caractère limitatif.

### EXEMPLES

### EXEMPLE DE PREPARATION A

Synthèse de Tosylate du 1H-2,6-diméthylpyrazolo [1,5-b]-1,2,4-triazole de structure:

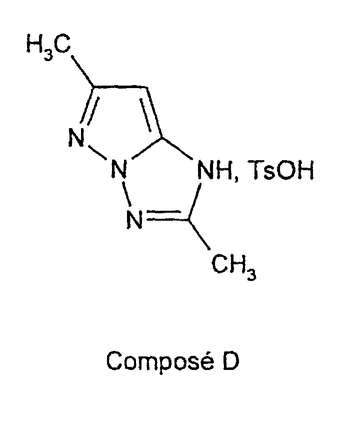

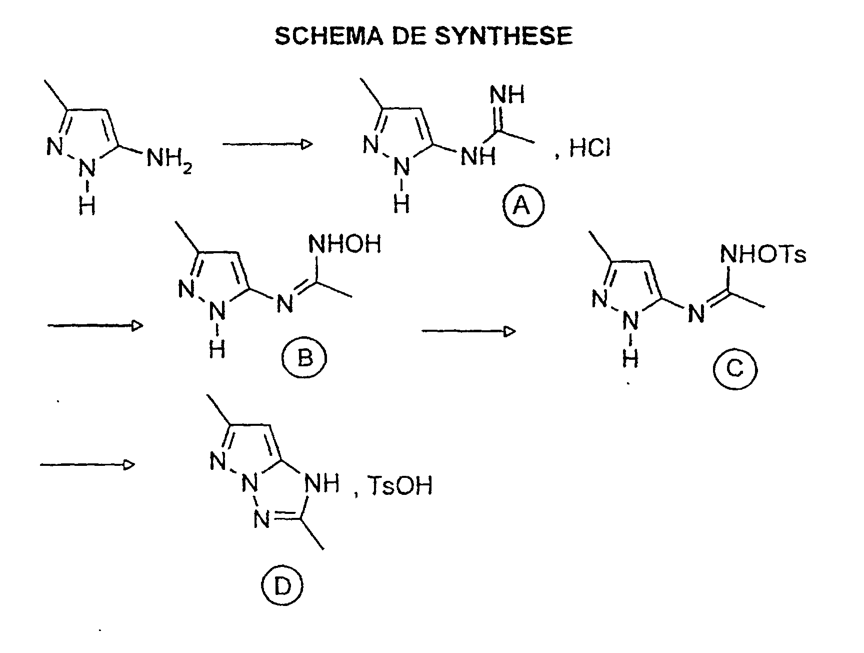

A une solution de 20 g (0,16 mole) de chlorhydrate d'éthylacétimidate dans 100 cm³ d'éthanol anhydre, on a ajouté, par petites portions, 15,7 g (0,16 mole) de 5-amino 3-méthylpyrazole sous agitation en maintenant la température en dessous de 30°C. L'agitation a été maintenue 1 heure supplémentaire. Le milieu réactionnel a ensuite été concentré à 45°C sous un vide de 60 mm de mercure jusqu'à début de cristallisation. On a refroidi à température ambiante et le précipité a été essoré sur un verre fritté puis séché sous vide à une température de 45°C. On a ainsi obtenu 16 g de composé A sous la forme de cristaux blancs dont le point de fusion était de 249°C.

A une solution de 70 g (0,4 mole) de composé A, préparé comme ci-dessus, dans 300 cm³ de méthanol anhydre, on a ajouté lentement 83,5 cm³ de solution méthanolique 4,8 M (0,4 mole) de méthylate de sodium. Il s'est formé du chlorure de sodium qui a été séparé par filtration. Le filtrat a été refroidi à 0°C et coulé dans une solution d'hydroxylamine elle-même refroidie à 0°C qui avait été précédemment préparée à partir de 33,4 g (0,48 mole) de chlorhydrate d'hydroxylamine, 20 cm³ de méthanol anhydre et 100 cm³ de solution de méthanolate de sodium 4,8 M (0,48 mole), le chlorure de sodium formé ayant été séparé par filtration.
Le milieu réactionnel a ensuite été chauffé en reflux, sous agitation pendant 24 heures, puis refroidi à environ 0°C. Un solide blanc a cristallisé. Il a été essoré sur un verre fritté, lavé avec du méthanol glacé et séché sous vide à 40°C. On a pesé 26,4 g de composé B. Le filtrat a été concentré sous vide et on a isolé de la même façon une seconde fraction de 7,6 g de composé B. Les 2 fractions ont été réunies, soit 34,3 g de composé B sous la forme d'un solide blanc dont le point de fusion était de 197°C.

A une solution de 20 g (0,13 mole) de composé B, préparé ci-dessus, dans 9,5 litres de tétrahydrofurane anhydre, on a ajouté 20,05 cm³ (0,143 mole) de triéthylamine anhydre puis 27,2 g (0,143 mole) du chlorure d'acide de l'acide paratoluènesulfonique. Le milieu réactionnel a été agité 2 heures à température ambiante puis refroidi à 0°C. Le chlorhydrate de triéthylamine a été séparé par filtration et le filtrat a été concentré sous un vide de 60 mm de mercure à environ 50°C jusqu'au début de la cristallisation. On a refroidi à 0°C et le précipité a été essoré sur un verre fritté, lavé avec du tétrahydrofurane glacé puis séché sous vide à une température de 40°C. On a ainsi obtenu 36,2 g de composé C sous la forme d'un solide blanc dont le point de fusion était compris entre 105°C et 128°C (décomposition).

On a chauffé pendant 2 heures au reflux une solution de 35 g (0,113 mole) de composé C obtenu ci-dessus dans 1 litre de méthanol puis on a évaporé à sec cette solution. On a obtenu une huile qui a cristallisé quand on a ajouté 100 cm³ d'éther isopropylique. Les cristaux ont été essorés sur un verre fritté puis recristallisés dans un mélange d'isopropanol et d'heptane. On a ainsi obtenu, après séchage sous vide à 40°C, 19 g de tosylate du 1H-2,6-diméthylpyrazolo [1,5-b]-1,2,4-triazole (composé D) sous la forme d'un solide blanc dont le point de fusion était de 157°C.

L'analyse élémentaire pour C₆H₉N₄, C₇H₇O₃S était :

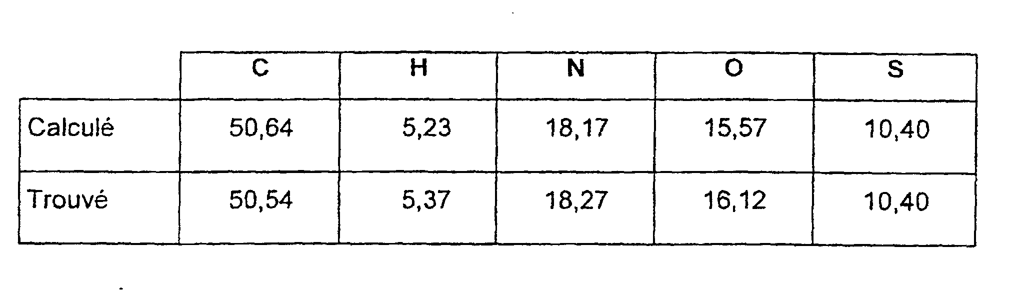

### EXEMPLES 1 à 5 DE TEINTURE EN MILIEU ALCALIN

On a préparé les compositions tinctoriales, conformes à l'invention, suivantes (teneurs en grammes) :

| **Exemple** | **1** | **2** | **3** | **4** | **5** |
|---|---|---|---|---|---|
| Composé de l'exemple de préparation A (coupleur) | 0,925 | 0,925 | 0,925 | 0,925 | 0,925 |
| Para-phénylènediamine (base d'oxydation) | 0,324 | - | - | - | - |
| Sulfate de paratoluylènediamine (base d'oxydation) | - | 0,660 | - | - | 0,805 |
| N-propyl para-phénylènediamine, sel de dichlorhydrate (base d'oxydation) | - | - | 0,669 | - | - |
| Para-aminophénol (base d'oxydation) | - | - | - | 0,327 | - |
| Dichlorhydrate de 1-méthyl 3-méthyl-diaminopyrazole (base d'oxydation) | - | - | - | - | 0,597 |
| Support de teinture commun | n°1 | n°1 | n°1 | n°1 | n°1 |
| Eau déminéralisée q.s.p. | 100 g | 100 g | 100 g | 100 g | 100 g |

### Support de teinture commun n°1 :

- Alcool oléique polyglycérolé à 2 moles de glycérol 4,0 g
- Alcool oléique polyglycérolé à 4 moles de glycérol à 78 % de matières actives (M.A.) 5,69 g M.A.
- Acide oléique 3,0 g
- Amine oléique à 2 moles d'oxyde d'éthylène vendue sous la dénomination commerciale ETHOMEEN 012 par la société AKZO 7,0 g
- Laurylamino succinamate de diéthylaminopropyle, sel de sodium à 55 % de M.A. 3,0 g M.A.
- Alcool oléique 5,0 g
- Diéthanolamide d'acide oléique 12,0 g
- Propylèneglycol 3,5 g
- Alcool éthylique 7,0 g
- Dipropylèneglycol 0,5 g
- Monométhyléther de propylèneglycol 9,0 g
- Métabisulfite de sodium à en solution aqueuse à 35 % de M.A. 0,455 g M.A.
- Acétate d'ammonium 0,8 g
- Antioxydant, séquestrant q.s.
- Parfum, conservateur q.s.
- Ammoniaque à 20 % de NH₃ 10,0 g

Au moment de l'emploi, chaque composition tinctoriale des exemples 1 à 5 a été mélangée avec un poids égal d'eau oxygénée à 20 volumes (6% en poids).

Chaque mélange obtenu a été appliqué pendant 30 minutes, sur des mèches de cheveux gris naturels à 90 % de blancs, permanentés ou non, à raison de 28 g pour 3 g de cheveux. Après rinçage, lavage avec un shampooing standard et séchage, les mèches ont été teintes dans les nuances figurant dans le tableau 1 ci-dessous :
La couleur des mèches a été évaluée dans le système MUNSELL, au moyen d'un colorimètre CM 2002 MINOLTA.

Selon la notation MUNSELL, une couleur est définie par l'expression **H V / C** dans laquelle les trois paramètres désignent respectivement la teinte ou Hue **(H)**, l'intensité ou Value **(V)** et la pureté ou Chromaticité **(C)**, la barre oblique de cette expression est simplement une convention et n'indique pas un ratio.

Les résultats sont donnés dans le tableau 1 ci-dessous :

**TABLEAU 1**

| **Exemple** | **Nuance obtenue sur cheveux gris naturels à 90% de blancs** | **Nuance obtenue sur cheveux gris à 90% de blancs permanentés** |
|---|---|---|
| **1** | Irisé violine | Irisé violine intense |
| | 8,5 RP 3,0/4,2 | 10,0 RP 2,6/4,0 |
| **2** | Irisé | Irisé intense |
| | 8,1 RP 3,4/5,3 | 8,8 RP 3,2/5,7 |
| **3** | Irisé | Irisé intense |
| | 8,2 RP 3,7/5,6 | 9,3 RP 3,1/5,9 |
| **4** | Irisé légèrement cuivré | Irisé cuivré |
| | 0,2 YR 4,7/2,9 | 0,7 YR 4,6/3,0 |
| **5** | Naturel doré cuivré | Doré cuivré |
| | 9,5 YR 5,3/3,4 | 8,9 YR 4,9/3,8 |

### EXEMPLES 6 à 9 DE TEINTURE EN MILIEU ALCALIN

On a préparé les compositions tinctoriales, conformes à l'invention, suivantes (teneurs en grammes):

| **Exemples** | **6** | **7** | **8** | **9** |
|---|---|---|---|---|
| 3,6-diméthyl- pyrazolo [3,2-c]-1,2,4-triazole (coupleur) | 0,408 | 0,408 | 0,408 | - |
| 6-phényl-3-méthylthio- pyrazolo [3,2-c]-1,2,4-triazole (coupleur) | - | - | - | 0,691 |
| Paratoluylènediamine (base d'oxydation) | 0,366 | - | - | 0,366 |
| Para-aminophénol (base d'oxydation) | - | 0,327 | - | - |
| Dichlorhydrate de 3,7-diamino-pyrazolo pyrimidine (base d'oxydation) | - | - | 0,666 | - |
| Support de teinture commun | n°2 | n°2 | n°2 | n°2 |
| Eau déminéralisée q.s.p. | 100 g | 100 g | 100 g | 100 g |
| **NB** : Le 3,6-diméthyl- pyrazolo [3,2-c]-1,2,4-triazole et le 6-phényl-3-méthylthio-pyrazolo [3,2-c]-1,2,4-triazole ont été préparés selon le procédé décrit dans J. Chem. Soc. Perkin trans 1, 2047, (1977). | | | | |

### Support de teinture commun n°2 :

- Ethanol 20 g
- Ammoniaque à 20% de NH₃ 10 g
- Métabisulfite de sodium 0,228 g
- Agent séquestrant q.s.

Au moment de l'emploi, chaque composition tinctoriale des exemples 6 à 9 ci-dessus a été mélangée avec un poids égal d'une solution de peroxyde d'hydrogène à 20 volumes (6% en poids).

Chaque mélange obtenu a été appliqué pendant 30 minutes, sur des mèches de cheveux gris naturels à 90 % de blancs, permanentés ou non, à raison de 10 g pour 1 g de cheveux. Après rinçage, lavage avec un shampooing standard et séchage, les mèches ont été teintes dans les nuances figurant dans le tableau 2 ci-dessous :

**TABLEAU 2**

| **Exemple** | **pH du mélange** | **Nuance obtenue sur cheveux gris naturels à 90% de blancs** | **Nuance obtenue sur cheveux gris à 90% de biancs permanentés** |
|---|---|---|---|
| **6** | 10,0 | Rouge irisé | Rouge irisé |
| **7** | 10,1 | Irisé cuivré | Cuivré irisé |
| **8** | 9,8 | Cuivré doré | Cuivré |
| **9** | 10,2 | Violine | Violine |

### EXEMPLES 10 et 11 DE TEINTURE EN MILIEU ALCALIN

On a préparé les compositions tinctoriales, conformes à l'invention, suivantes (teneurs en grammes) :

| **Exemples** | **10** | **11** |
|---|---|---|
| 6-méthyl- pyrazolo [1,5-a] benzimidazole (coupleur) | 0,514 | - |
| 7-chloro-2,6-diméthylpyrazolo[1,5-b]-1,2,4-triazole (coupleur) | - | 0,512 |
| Paraphénylènediamine (base d'oxydation) | 0,324 | 0,324 |
| Support de teinture commun | n°3 | n°3 |
| Eau déminéralisée q.s.p. | 100 g | 100 g |
| **NB** : Le 6-méthyl- pyrazolo [1,5-a] benzimidazole a été préparé selon le procédé décrit dans J. fur Chem., 326(5), 829, (1984), et le 7-chloro-2,6-diméthylpyrazolo[1,5-b]-1,2,4-triazole a été préparé selon le procédé décrit dans la demande de brevet EP-A-119 860. | | |

### Support de teinture commun n°3

- Alcool benzylique 2,0 g
- Polyéthylène glycol à 6 moles d'oxyde d'éthylène 3,0 g
- Ethanol 20,0 g
- Alkyl (C₈-C₁₀) polyglucoside en solution aqueuse à 60% de matière active, tamponné par du citrate d'ammonium, vendu sous la dénomination ORAMIX CG110 par la société SEPPIC 6,0 g
- Ammoniaque à 20% de NH₃ 10,0 g
- Métabisulfite de sodium 0,228 g
- Agent séquestrant q.s.

Au moment de l'emploi, chaque composition tinctoriale des exemples 10 et 11 ci-dessus a été mélangée avec un poids égal d'une solution de peroxyde d'hydrogène à 20 volumes (6% en poids). Chaque mélange obtenu a été appliqué pendant 30 minutes, sur des mèches de cheveux gris naturels à 90 % de blancs, permanentés ou non, à raison de 10g pour 1 g de cheveux. Après rinçage, lavage avec un shampooing standard et séchage, les mèches ont été teintes dans les nuances figurant dans le tableau 3 ci-dessous :

**TABLEAU 3**

| **Exemple** | **pH du mélange** | **Nuance obtenue sur cheveux gris naturels à 90% de blancs** | **Nuance obtenue sur cheveux gris à 90% de blancs permanentés** |
|---|---|---|---|
| **10** | 10,1 | irisé rabattu | Irisé légèrement violine |
| **11** | 10,1 | Irisé | Irisé légèrement rouge |

### EXEMPLE 12 DE TEINTURE EN MILIEU ALCALIN

La même composition tinctoriale que celle utilisée pour l'exemple 10 ci-dessus a été préparée. Au moment de l'emploi, cette composition tinctoriale a été mélangée avec une quantité égale en poids d'une solution aqueuse de persulfate d'ammonium à 6.10⁻³ mole %. Le mélange obtenu présentait un pH de 9,9 et a été appliqué pendant 30 minutes, sur des mèches de cheveux gris naturels à 90 % de blancs, permanentés ou non, à raison de 10 g pour 1 g de cheveux. Après rinçage, lavage avec un shampooing standard et séchage, les mèches ont été teintes dans une nuance doré irisé sur cheveux naturels, et dans une nuance irisé légèrement rouge sur cheveux permanentés.

### EXEMPLES 13 à 17 DE TEINTURE EN MILIEU NEUTRE

On a préparé les compositions tinctoriales, conformes à l'invention, suivantes (teneurs en grammes) :

| **Exemples** | **13** | **14** | **15** | **16** | **17** |
|---|---|---|---|---|---|
| 3,6-diméthyl- pyrazolo [3,2-c]-1,2,4-triazole (coupleur) | 0,408 | 0,408 | - | - | - |
| 6-phényl-3-méthylthiopyrazolo [3,2-c]-1,2,4-triazole (coupleur) | - | - | 0,691 | - | - |
| 7-chloro-2,6-diméthylpyrazolo[1,5-b]-1,2,4-triazole (coupleur) | - | - | - | 0,512 | - |
| 6-méthylpyrazolo[5,1e] tétrazole (coupleur) | - | - | - | - | 0,370 |
| Paratoluylènediamine (base d'oxydation) | 0,366 | - | - | - | - |
| Dichlorhydrate de 3,7-diamino-pyrazolo pyrimidine (base d'oxydation) | - | 0,666 | 0,666 | - | - |
| Paraphénylènediamine (base d'oxydation) | - | - | - | 0,324 | - |
| Dichlorhydrate de 4-(2-méthoxyéthylamino)aniline (base d'oxydation) | - | - | - | - | 0,717 |
| Support de teinture commun | n°4 | n°4 | n°4 | n°5 | n°4 |
| Eau déminéralisée q.s.p. | 100 g | 100 g | 100 g | 100 g | 100 g |
| **NB** : Le 6-méthylpyrazolo[5,1e] tétrazole a été préparé selon le procédé décrit dans le brevet JP 60 33 552. | | | | | |

### Support de teinture commun n°4 :

- Ethanol 20,0 g
- Tampon K₂HPO₄/KH₂PO₄ (1.5 M / 1 M) 10,0 g
- Métabisulfite de sodium 0,228 g
- Agent séquestrant q.s.

### Support de teinture commun n°5 :

- Alcool benzylique 2,0 g
- Polyéthylène glycol 6 OE 3,0 g
- Ethanol 20,0 g
- Alkyl (C₈-C₁₀) polyglucoside en solution aqueuse à 60% de matière active tamponné par du citrate d'ammonium vendu sous la dénomination ORAMIX CG110 par la société SEPPIC 6,0 g
- Tampon K₂HPO₄ / KH₂PO₄ (1.5 M/1 M) 10,0 g
- Métabisulfite de sodium 0,228 g
- Agent séquestrant q.s.

Au moment de l'emploi, chaque composition tinctoriale des exemples 13 à 17 ci-dessus a été mélangée avec un poids égal d'une solution de peroxyde d'hydrogène à 20 volumes (6% en poids).

Chaque mélange obtenu a été appliqué pendant 30 minutes, sur des mèches de cheveux gris naturels à 90 % de blancs, permanentés ou non, à raison de 10 g pour 1 g de cheveux. Après rinçage, lavage avec un shampooing standard et séchage, les mèches ont été teintes dans les nuances figurant dans le tableau 4 ci-dessous :

**TABLEAU 4**

| **Exemple** | **pH du mélange** | **Nuance obtenue sur cheveux gris naturels à 90% de blancs** | **Nuance obtenue sur cheveux gris à 90% de blancs permanentés** |
|---|---|---|---|
| **13** | 7,1 | Irisé rouge | Rouge irisé |
| **14** | 6,9 | Cuivré doré rabattu | Cuivré |
| **15** | 6,7 | Cuivré irisé | Cuivré rouge |
| **16** | 6,8 | Violine | Violine profond |
| **17** | 6,8 | Gris bleuté violacé | Gris bleuté |

## Revendications

1. Composition pour la teinture des fibres kératiniques et en particulier des fibres kératiniques humaines telles que les cheveux, **caractérisée par le fait qu'**elle comprend, dans un milieu approprié pour la teinture :
- à titre de coupleur, au moins un composé pyrazolo-azole de formule (I) suivante ou l'un de ses sels d'addition avec un acide : 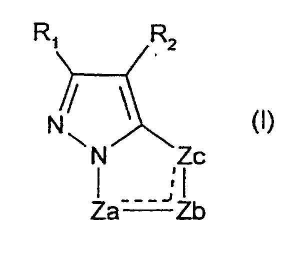 dans laquelle :
· R₁ représente : un atome d'hydrogène ; un radical alkyle en C₁-C₂₀, linéaire ou ramifié, éventuellement substitué par 1 ou 2 radicaux R choisis dans le groupe constitué par halogène, nitro, cyano, hydroxy, alcoxy, aryloxy, amino, alkylamino, acylamino, carbamoyle, sulfonamido, sulfamoyle, imido, alkylthio, arylthio, aryle, alcoxycarbonyle, acyle ; un radical aryle éventuellement substitué par 1 ou 2 radicaux R tels que précédemment définis ; un hétérocycle à 5 ou 6 chaînons possédant au moins un atome d'azote, d'oxygène ou de soufre, éventuellement substitué par 1 ou 2 radicaux R tels que définis précédemment ;
lorsque R₁ désigne un radical alkyle, un radical aryle ou un hétérocycle à 5 ou 6 chaînons (définis ci-dessus), il peut être relié à l'atome de carbone du noyau par l'intermédiaire d'un atome d'oxygène, d'azote ou de soufre (dans ce cas, R₁ devient XR₁ avec X = O, NH, S) ;
lorsque R₁ désigne un radical alkyle, un radical aryle ou un hétérocycle à 5 ou 6 chaînons (définis ci-dessus), il peut être relié à l'atome de carbone du noyau par l'intermédiaire d'un atome d'oxygène, d'azote ou de soufre (dans ce cas, R₁ devient XR₁ avec X = O, NH, S) ;
lorsque R₁ désigne un radical alkyle, un radical aryle ou un hétérocycle à 5 ou 6 chaînons (définis ci-dessus), il peut être relié à l'atome de carbone du noyau par l'intermédiaire d'un atome d'oxygène, d'azote ou de soufre (dans ce cas, R₁ devient XR₁ avec X = O, NH, S) ;
R₁ peut désigner aussi un atome d'halogène ; un radical acyle ; un radical sulfonyle ; un radical sulfinyle ; un radical phosphonyle, un radical carbamoyle ; un radical sulfamoyle ; un radical cyano ; un radical siloxy, un radical amino ; un radical acylamino ; un radical acyloxy ; un radical carbamoyloxy ; un radical sulfonamide ; un radical imide ; un radical uréido ; un radical sulfamoylamino ; un radical alcoxycarbonylamino ; un radical aryloxycarbonylamino ; un radical alcoxycarbonyle ; un radical aryloxycarbonyle ; un radical carboxyle ;
· R₂ représente : un atome d'hydrogène ; un atome d'halogène ; un radical acétylamido ; un radical alcoxy ; un radical aryloxy ; un radical acyloxy ; un radical arylthio ; un radical alkylthio ;un radical hétéroarylthio ; un radical hétéroaryloxy ; un radical thiocyano ; un radical N,N-diéthyl thiocarbonylthio ; un radical dodécyloxythio carbonylthio ; un radical benzène-sulfonamîdo ; un radical N-éthyltoluène sulfonamido ; un radical pentafluorobutanamido ; un radical 2,3,4,5,6-penta-fluorobenzamido ; un radical p-cyanophényluréido, un radical N,N-diéthyl-sulfamoylamino ; un radical pyrazolyle ; un radical imidazolyle ; un radical triazolyle ; un radical tétrazolyle ; un radical benzimidazolyle ; un radical 1-benzyl 5-éthoxy 3-hydantoïnyle ; un radical 1-benzyl 3-hydantoïnyle ; 5,5-diméthyl 2,4-dioxo 3-oxazolidinyle ; un radical 2-oxy 1,2-dihydro 1-pyridinyle ; un alkylamido ; un arylamido; un radical NR^{III}R^{IV} avec R^{III} et R^{IV} représentant, identiques ou différents, un alkyle en C₁-C₄, un hydroxyalkyle ; un carboxyle ; un radical alcoxycarboxylique.
Zₐ, Z_{b}, Z_{c} représentent, indépendamment l'un de l'autre, un atome d'azote, un atome de carbone portant un radical R₃ ou R₄ ayant les mêmes significations que celles indiquées pour le radical R₁ ; sous réserve que l'un au moins des radicaux Zₐ, Z_{b} et Z_{c} soit différent d'un atome de carbone ; R₃ et R₄ peuvent également former entre eux un cycle aromatique substitué ou non ;
· et au moins une base d'oxydation.

2. Composition selon la revendication 1, **caractérisée par le fait que** les radicaux R₁ de la formule (I) sont choisis dans le groupe constitué par :
un atome d'hydrogène ; un alkyle en C₁-C₄, linéaire ou ramifié ; un phényle ; un phényle substitué par un atome d'halogène, un alkyle en C₁-C₄, un alcoxy en C₁-C₄, un groupe nitro, un groupe amino, un groupe trifluorométhyle ou alkylamino en C₁-C₄ ; un radical benzyle ; un radical benzyle substitué par un atome d'halogène, un alkyle en C₁-C₄, un alcoxy en C₁-C₄, un groupe nitro, un groupe amino, un groupe trifluorométhyle ; un alkylamino en C₁-C₄ ; un hétérocycle choisi parmi le thiophène, le furane ou la pyridine ; un radical trifluorométhyle ; un radical (CH₂)ₚ-X-(CH₂)_{q}-OR' où p et q sont entiers, identiques ou différents, compris entre 1 et 3, R' représente H ou méthyle et X désigne un atome d'oxygène ou un groupe NR" avec R" désignant hydrogène ou méthyle ; un hydroxyalkyle en C₁-C₄ ; un aminoalkyle en C₁-C₄ ; un alkylamino en C₁-C₄ ; un dialkylamino en C₁-C₄ ; un arylamino ; un radical alcoxy choisi parmi méthoxy, éthoxy et phénoxy ; un halogène choisi parmi fluor, chlore et brome ; un groupe carboxyle ; un alcoxycarbonyle en C₁-C₄ ; un phényloxycarbonyle ; méthylthio ; éthylthio ; phénylthio ; méthanesulfonyle ; cyano.

3. Composition selon la revendication 1 ou 2, **caractérisée par le fait que** les radicaux R₁ de la formule (I) sont choisis dans le groupe constitué par :
hydrogène ; un alkyle choisi parmi méthyle, éthyle, isopropyle, ter-butyle ; un halogène choisi parmi fluor et chlore ; phényle ; toluyle ; 4-chlorophényle ; 4-méthoxyphényle ; 3-méthoxyphényle ; 2-mèthoxyphényle ; benzyle ; un hétérocycle choisi parmi pyridyle, furyle ou thiènyle ; trifluorométhyle ; hydroxyméthyle ; aminométhyle ; méthoxy ou éthoxy ; méthylamino ou éthylamino ou diméthylamino ; carboxyle ; méthoxycarbonyle ou éthoxycarbonyle ; cyano.

4. Composition selon la revendication 3, **caractérisée par le fait que** les radicaux R₁ de la formule (I) sont choisis dans le groupe constitué par :
hydrogène ; méthyle ; éthyle ; phényle ; toluyle ; 4-chlorophényle ; 4-méthoxyphényle ; benzyle ; trifluorométhyle ; chloro ; un radical méthoxy ou éthoxy ; un radical carboxyle ; un radical méthylamino ou diméthylamino ; cyano.

5. Composition selon l'une quelconque des revendications 1 à 4, **caractérisée par le fait que** les radicaux R₂ de la formule (I) sont choisis dans le groupe constitué par :
un atome d'hydrogène ; un alcoxy en C₁-C₄ ; phénoxy ; phénoxy substitué par un atome d'halogène, un alkyle en C₁-C₄, un carboxyle, un groupe trifluorométhyle ; un radical acyloxy ; benzyloxy ; alkylthio en C₁-C₄ ; phénylthio ; phénylthio substitué par un atome d'halogène, un alkyle en C₁-C₄, un carboxyle, un groupe trifluorométhyle ; un alkylamido en C₁-C₄ ; phénylamido ; un radical NR^{III}R^{IV} avec R^{III} et R^{IV} représentant, identiques ou différents, un alkyle en C₁-C₄ , un hydroxyalkyle en C₁-C₄ ; un carboxyle ; un radical alcoxycarboxylique en C₁-C₄.

6. Composition selon la revendication 5, **caractérisée par le fait que** les radicaux R₂ de la formule (I) sont choisis dans le groupe constitué par :
hydrogène ; chlore ou brome ; méthoxy ou éthoxy ; phényloxy ; 4-méthylphényloxy ; acyloxy ; benzyloxy ; méthylthio ou éthylthio ; phénylthio ; 4-méthylphénylthio ; 2-tertio-butylphénylthio ; acétamido ; phénylacétamido ; diméthylamino ; diéthylamino ; éthylméthylamino ; (β-hydroxyéthyl)méthylamino.

7. Composition selon la revendication 6, **caractérisée par le fait que** les radicaux R₂ de la formule (I) sont choisis dans le groupe constitué par :
hydrogène ; chlore ; éthoxy ; phénoxy ; benzyloxy ; acyloxy ; acétamido ; diméthylamino.

8. Composition selon l'une quelconque des revendications 1 à 7, **caractérisée par le fait que** les radicaux R₃ et R₄ de la formule (I) sont choisis dans le groupe constitué par :
un atome d'hydrogène.; un alkyle en C₁-C₄, linéaire ou ramifié ; un trifluorométhyle ; un phényle ; un phényle substitué par un ou deux groupes choisis parmi un halogène, un alkyle en C₁-C₄, un alcoxy en C₁-C₄, un hydroxy, un carboxyle, un groupe nitro, un alkylthio en C₁-C₄, un groupe méthylènedioxy, un groupe amino, un groupe trifluorométhyle ou un alkylamino en C₁-C₄ ; un radical benzyle ; un radical benzyle substitué par un atome d'halogène, un méthyle ou isopropyle, méthoxy ; un hydroxyalkyle en C₁-C₄ ; un aminoalkyle en C₁-C₄ ; un alkylaminoalkyle en C₁-C₄ ; un radical alcoxy choisi parmi méthoxy, éthoxy et phénoxy ; méthylthio ; éthylthio ; phénylthio ; méthanesulfonyle ; un cycle aromatique substitué ou non formé par R₃ et R₄.

9. Composition selon la revendication 8, **caractérisée par le fait que** les radicaux R₃ et R₄ de la formule (I) sont choisis dans le groupe constitué par :
hydrogène ; un alkyle choisi parmi méthyle, éthyle, isopropyle, n-propyle, ter-butyle ; phényle ; toluyle ; 2-, 3- ou 4-chlorophényle ; 3- ou 4-hydroxyphényle ; 3- ou 4-aminophényle ; 3- ou 4-méthoxyphényle ; 4-trifiuorométhylphényle ; benzyle ; trifluorométhyle ; hydroxyméthyle ; hydroxyéthyle ; hydroxyisopropyle ; aminométhyle ou aminoéthyle ; méthoxy ou éthoxy ; méthylthio ou éthylthio ; un cycle phényle formé par R₃ et R₄ tel que phényle.

10. Composition selon la revendication 9, **caractérisée par le fait que** les radicaux R₃ et R₄ de la formule (I) sont choisis dans le groupe constitué par :
hydrogène ; méthyle ; éthyle ; isopropyle ; phényle ; 4-chlorophényle ; 4-méthoxy-phényle ; 4-aminophényle ; méthoxy ou éthoxy ; méthylthio ou éthylthio ; un cycle phényle formé par R₃ et R₄.

11. Composition selon l'une quelconque des revendications 1 à 10, **caractérisée par le fait que** les composés de formule (I) sont choisis dans le groupe constitué par :
(i) les pyrazolo-[1,5-b]-1,2,4-triazoles de formule : 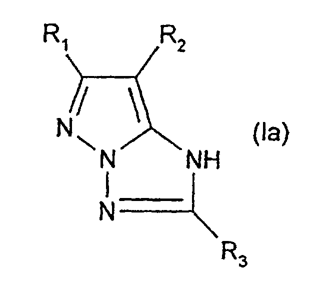
(ii) les pyrazolo [3,2-c] 1,2,4-triazoles de formule : 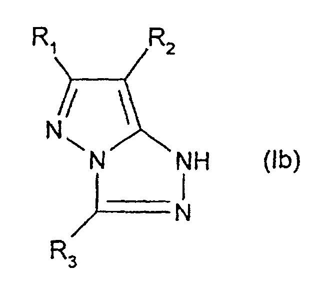
(iii) les pyrazolo tétrazoles de formule : 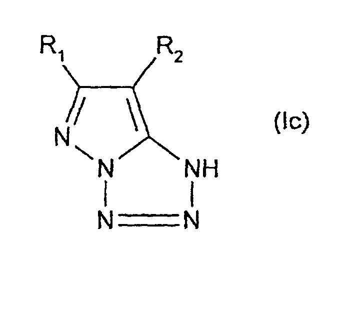
(iv) les pyrazolo-[1, 5-a]-imidazoles de formule : 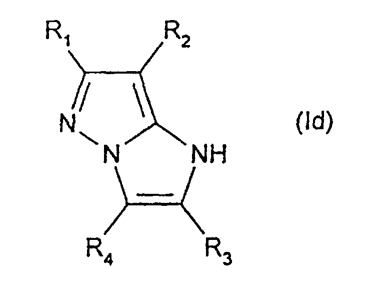
(v) les pyrazolo-[5, 1-e]-pyrazoles de formule : 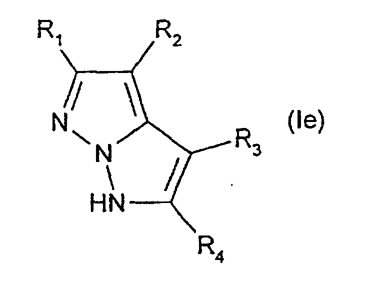
(vi) les pyrazolo-[5,1-e]-1,2,3-triazoles de formule : 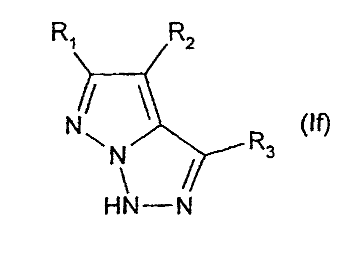
dans lesquelles R₁, R₂, R₃ et R₄ ont les mêmes significations que celles indiquées dans l'une quelconque des revendications 1 à 10 ; ainsi que leurs sels d'addition avec un acide.

12. Composition selon la revendication 11, **caractérisée par le fait que** les composés de formule (la) ou (lb) sont choisis parmi ceux pour lesquels :
- R₁ désigne hydrogène, méthyle, éthylthio, amino, trifluorométhyle, carboxyle, phényle, éthoxy ou cyano
- R₂ désigne hydrogène ou chlore ;
- R₃ hydrogène, méthyle, éthyle, isopropyle, β-aminoéthyle, β-hydroxyéthyle, phényle, méthylthio ou éthoxy.

13. Composition selon la revendication 11, **caractérisée par le fait que** les composés de formule (lc) sont choisis parmi ceux pour lesquels :
- R₁, désigne hydrogène, méthyle, trifluorométhyle, carboxyle, phényle, éthoxy ou cyano
- R₂ désigne hydrogène ou chlore.

14. Composition selon la revendication 11, **caractérisée par le fait que** les composés de formule (ld) sont choisis parmi ceux pour lesquels :
- R₁ désigne hydrogène, méthyle, trifluorométhyle, amino, carboxyle, phényle, éthoxy ou cyano
- R₂ désigne hydrogène ou chlore ;
- R₃ et R₄ désignent respectivement hydrogène et hydrogène, hydrogène et méthyle, méthyle et hydrogène, hydrogène et amino, hydrogène et phényle ; ou forment entre eux un cycle phényle.

15. Composition selon la revendication 11, **caractérisée par le fait que** les composés de formule (le) sont choisis parmi ceux pour lesquels :
- R₁ désigne hydrogène, méthyle, trifluorométhyle, carboxyle, phényle, éthoxy ou cyano
- R₂ désigne hydrogène ou chlore ;
- R₃ et R₄ désignent respectivement hydrogène et méthyle, méthyle et hydrogène, méthyle et méthyle, hydrogène et phényle.

16. Composition selon la revendication 11, **caractérisée par le fait que** les composés de formule (If) sont choisis parmi ceux pour lesquels :
- R₁ désigne hydrogène, méthyle, trifluorométhyle, carboxyle, phényle, éthoxy ou cyano
- R₂ désigne hydrogène ou chlore ;
- R₃ hydrogène ou méthyle.

17. Composition selon la revendication 11, **caractérisée par le fait que** les composés de formule (I) sont choisis parmi :
- le 2-méthyl pyrazolo [1,5-b]-1,2,4-triazole,
- le 2-éthyl pyrazolo [1,5-b]-1,2,4-triazole,
- le 2-isopropyl pyrazolo [1,5-b]-1,2,4-triazole,
- le 2-phényl pyrazolo [1,5-b]-1,2,4-triazole,
- le 2,6-diméthyl pyrazolo [1,5-b]-1,2,4-triazole,
- le 6-méthyl-2-éthyl pyrazolo [1,5-b]-1,2,4-triazole,
- le 6-méthyl-2-isopropyl pyrazolo [1,5-b]-1,2,4-triazote,
- le 6-méthyl-2-phényl pyrazolo [1,5-b]-1,2,4-triazole,
- le 6-carboxy-2-méthyl pyrazolo [1,5-b]-1,2,4-triazole,
- le 6-carboxy-2-éthyl pyrazolo [1,5-b]-1,2,4-triazole,
- le 6-carboxy-2-isopropyl pyrazolo [1,5-b]-1,2,4-triazole,
- le 6-carboxy-2-phényl pyrazolo [1,5-b]-1,2,4-triazole,
- le 6-phényl-2-méthyl pyrazolo [1,5-b]-1,2,4-triazole,
- le 6-phényl-2-éthyl pyrazolo [1,5-b]-1,2,4-triazole,
- le 6-phényl-2-isopropyl pyrazolo [1,5-b]-1,2,4-triazole,
- le 6-phényl-2-phényl pyrazolo [1,5-b]-1,2,4-triazole,
- le 6-amino-2-méthyl pyrazolo [1,5-b]-1,2,4-triazole,
- le 6-amino-2-éthyl pyrazolo [1,5-b]-1,2,4-triazole,
- le 6-amino-2-isopropyl pyrazolo [1,5-b]-1,2,4-triazole,
- le 6-amino-2-phényl pyrazolo [1,5-b]-1,2,4-triazole,
- le 6-éthylthio-2-méthyl pyrazolo [1,5-b]-1,2,4-triazole,
- le 6-éthylthio-2-éthyl pyrazolo [1,5-b]-1,2,4-triazole,
- le 6-éthylthio-2-isopropyl pyrazolo [1,5-b]-1,2,4-triazole,
- le 6-éthylthio-2-phényl pyrazolo [1,5-b]-1,2,4-triazole,
- le 6-éthoxy-2-méthyl pyrazolo [1,5-b]-1,2,4-triazole,
- le 6-éthoxy-2-éthyl pyrazolo [1,5-b]-7,2,4-triazole,
- le 6-éthoxy-2-isopropopyl pyrazolo [1,5-b]-1,2,4-triazole,
- le 6-éthoxy-2-phényl pyrazolo [1,5-b]-1,2,4-triazole,
- le 6-méthyl-2-(2'-aminoéthyl) pyrazolo [1,5-b]-1,2,4-triazole,
- le 6-carboxy-2-(2'-aminoéthyl) pyrazolo [1,5-b]-1,2,4-triazole,
- le 6-phényl-2-(2'-aminoéthyl) pyrazolo [1,5-b]-1,2,4-triazole,
- le 6-éthylthio-2-(2'-aminoéthyl) pyrazolo [1,5-b]-1,2,4-triazole,
- le 2-(2'-aminoéthyl) pyrazolo [1,5-b]-1,2,4-triazole,
- le 2-(2'-hydroxyéthyl) pyrazolo [1,5-b]-1,2,4-triazole,
- le 6-méthyl-2-(2'-hydroxyéthyl) pyrazolo [1,5-b]-1,2,4-triazole,
- le 6-éthylthio-2-(2'-hydroxyéthyl) pyrazolo [1,5-b]-1,2,4-triazole,
- le 6-carboxy-2-(2'-hydroxyéthyl) pyrazolo [1,5-b]-1,2,4-triazole,
- le 6-phényl-2-(2'-hydroxyéthyl) pyrazolo [1,5-b]-1,2,4-triazole,
- le 7-chloro-2,6-diméthylpyrazolo[1,5-b]-1,2,4-triazole,
- le 7-bromo -2,6-diméthylpyrazolo[1,5-b]-1,2,4-triazole,
- le 3-méthyl pyrazolo [3,2-c]-1,2,4-triazole,
- le 3-méthylsulfinyl-6-phényl-pyrazolo [3,2-c]-1,2,4-triazole,
- le 3-éthyl pyrazolo [3,2-c]-1,2,4-triazole,
- le 3-isopropyl pyrazolo [3,2-c]-1,2,4-triazole,
- le 3-phényl pyrazolo [3,2-c]-1,2,4-triazole,
- le 3-(2'-aminoéthyl) pyrazolo [3,2-c]-1,2,4-triazole,
- le 3-(2'-hydroxyéthyl) pyrazolo [3,2-c]-1,2,4-triazole,
- le 6-méthyl-3-éthyl pyrazolo [3,2-c]-1,2,4-triazole,
- le 3,6-diméthyl- pyrazolo [3,2-c]-1,2,4-triazole,
- le 6-méthyl-3-isopropyl- pyrazolo [3,2-c]-1,2,4-triazole,
- le 6-méthyl-3-phényl- pyrazolo [3,2-c]-1,2,4-triazole,
- le 6-méthyl-3-(2'-aminoéthyl)- pyrazolo [3,2-c]-1,2,4-triazole,
- le 6-méthyl-3-(2'hydroxyéthyl)- pyrazolo [3,2-c]-1,2,4-triazole,
- le 6-méthyl-3-méthylthio- pyrazolo [3,2-c]-1,2,4-triazole,
- le 6-phényl-3-méthyl- pyrazolo [3,2-c]-1,2,4-triazole,
- le 6-phényl-3-éthyl- pyrazolo [3,2-c]-1,2,4-triazole,
- le 6-phényl-3-isopropyl- pyrazolo [3,2-c]-1,2,4-triazole,
- le 6-phényl-3-phényl- pyrazolo [3,2-c]-1,2,4-triazole,
- le 6-phényl-3-(2'-aminoéthyl)-pyrazolo [3,2-c]-1,2,4-triazole,
- le 6-phényl-3-(2'-hydroxyéthyl)- pyrazolo [3,2-c]-1,2,4-triazole,
- le 6-phényl-3-méthylthio- pyrazolo [3,2-c]-1,2,4-triazole,
- le 6-éthylthio-3-méthyl- pyrazolo [3,2-c]-1,2,4-triazole,
- le 6-éthylthio-3-éthyl- pyrazolo [3,2-c]-1,2,4-triazole,
- le 6-éthylthio-3-isopropyl- pyrazolo [3,2-c]-1,2,4-triazole,
- le 6-éthylthio-3-phényl- pyrazolo [3,2-c]-1,2,4-triazole,
- le 6-éthylthio-3-(2'-aminoéthyl)- pyrazolo [3,2-c]-1,2,4-triazole,
- le 6-éthylthio-3-(2'-hydroxyéthyl)- pyrazolo [3,2-c]-1,2,4-triazole,
- le 6-trifluorométhyl-3-méthylthio- pyrazolo [3,2-c]-1,2,4-triazole,
- le 6-trifluorométhyl- pyrazolo [3,2-c]-1,2,4-triazole,
- le 6-carboxy-3-méthyl- pyrazolo [3,2-c]-1,2,4-triazole,
- le 6-carboxy-3-éthyl- pyrazolo [3,2-c]-1,2,4-triazole,
- le 6-carboxy-3-isopropyl- pyrazolo [3,2-c]-1,2,4-triazole,
- le 6-carboxy-3-phényl- pyrazolo [3,2-c]-1,2,4-triazole,
- le 6-carboxy-3-(2'amino-éthyl)- pyrazolo [3,2-c]-1,2,4-triazole,
- le 6-carboxy-3-(2'-hydroxyéthyl)- pyrazolo [3,2-c]-1,2,4-triazole,
- le 7-chloro-3,6-diméthylpyrazolo[3,2-c]-1,2,4-triazole,
- le 7-méthoxycarbonyl-3,6-diméthylpyrazolo [3,2-c]-1,2,4-triazole,
- le pyrazolo [5,1-e]-tétrazole,
- le 6-méthyl pyrazolo [5,1-e]-tétrazole,
- le 6-phényl pyrazolo [5,1-e]-tétrazole,
- le 6-carboxy pyrazolo [5,1-e]-tétrazole,
- le 7-chloro-6-méthyl pyrazolo [5,1-e]-tétrazole,
- le pyrazolo [1,5-a] imidazole,
- le 2-méthyl- pyrazolo [1,5-a] imidazole,
- le 2-phényl - pyrazolo [1,5-a] imidazole,
- le pyrazolo [1,5-a] benzimidazole,
- le 6-méthyl- pyrazolo [1,5-a] imidazole,
- le 2,6-diméthyl- pyrazolo [1,5-a] imidazole,
- le 6-méthyl-2-phényl- pyrazolo [1,5-a] imidazole,
- le 6-méthyl- pyrazolo [1,5-a] benzimidazole,
- le 6-phényl- pyrazolo [1,5-a] imidazole,
- le 6-phényl-2-méthyl- pyrazolo [1,5-a] imidazole,
- le 2,6-diphényl- pyrazolo [1,5-a] imidazole,
- le 6-phényl- pyrazolo [1,5-a] benzimidazole,
- le 6-carboxy- pyrazolo [1,5-a] imidazole,
- le 6-carboxy-2-méthyl- pyrazolo [1,5-a] imidazote,
- le 6-carboxy-2-phényl- pyrazolo [1,5-a] imidazole,
- le 6-carboxy- pyrazolo [1,5-a] benzimidazole,
- le 6-éthoxy- pyrazolo [1,5-a] imidazole,
- le 6-éthoxy-2-méthyl- pyrazolo [1,5-a] imidazole,
- le 6-éthoxy-2-phényl- pyrazolo [1,5-a] imidazole,
- le 6-trifluorométhyl- pyrazolo [1,5-a] benzimidazole,
- le 6-amino- pyrazolo [1,5-a] imidazole,
- le 6-amino-2-méthyl- pyrazolo [1,5-a] imidazole,
- le 6-amino-2-phényl - pyrazolo [1,5-a] imidazole,
- le 6-amino- pyrazolo [1,5-a] benzimidazole,
- le 6-éthylthio- pyrazolo [1,5-a] imidazole,
- le 6-éthylthio-2-méthyl- pyrazolo [1,5-a] imidazole,
- le 6-éthylthio-2-phényl- pyrazolo [1,5-a] imidazole,
- le 7-chloro -6-méthyl- pyrazolo [1,5-a] imidazole,
- le 7-chloro -6-méthyl- pyrazolo [1,5-a] benzimidazole,
- le 8-amino-4-méthyl-pyrazolo [5,1-e]- pyrazole,
- le 8-amino-5-chloro-4-méthyl-pyrazolo [5,1-e]- pyrazole,
- le 5-méthylpyrazolo [5,1-e]-1, 2, 3 triazole,
- le 5-méthyl-6-chloro- pyrazolo [5,1-e]-1, 2, 3 triazole,
- le 5-phénylpyrazolo [5,1-e]-1, 2, 3 triazole,
et leurs sels d'addition avec un acide.

18. Composition selon l'une quelconque des revendications 1 à 11, **caractérisée par le fait qu'**elle contient au moins un composé de formule :

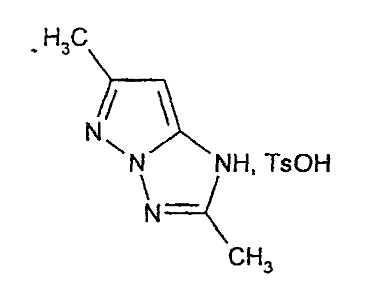

19. Composition selon l'une quelconque des revendications précédentes, **caractérisée par le fait que** les sels d'addition avec un acide des composés de formule (I) sont choisis parmi les chlorhydrates, les bromhydrates, les sulfates, les tartrates, les benzènesulfonates, les lactates, les tosylates et les acétates.

20. Composition selon l'une quelconque des revendications précédentes, **caractérisée par le fait que** le ou les composés de formule (I) représentent de 0,0005 à 12 % en poids du poids total de la composition tinctoriale.

21. Composition selon la revendication 20, **caractérisée par le fait que** le ou les composés de formule (I) représentent de 0,0005 à 12 % en poids du poids total de la composition tinctoriale.

22. Composition selon l'une quelconque des revendications précédentes, **caractérisée par le fait que** la ou les bases d'oxydation sont choisies parmi les paraphénylènediamines, les bis-phénylalkylènediamines, les para-aminophénols, les ortho-aminophénols, les bases hétérocycliques, et leurs sels d'addition avec un acide.

23. Composition selon l'une quelconque des revendications précédentes, **caractérisée par le fait que** la ou les bases d'oxydation représentent de 0,0005 à 12% en poids environ du poids total de la composition tinctoriale.

24. Composition selon la revendication 23, **caractérisée par le fait que** la ou les bases d'oxydation représentent de 0,005 à 6 % en poids environ du poids total de la composition tinctoriale.

25. Composition selon l'une quelconque des revendications précédentes, **caractérisée par le fait qu'**elle renferme en outre un ou plusieurs coupleurs additionnels différents des composés de formule (I) et/ou un ou plusieurs colorants directs.

26. Composition selon l'une quelconque des revendications précédentes, **caractérisée par le fait que** le milieu approprié pour la teinture (ou support) est constitué par de l'eau ou par un mélange d'eau et d'au moins un solvant organique choisi parmi les alcools inférieurs en C₁-C₄, le glycérol, les glycols et éthers de glycols, les alcools aromatiques, les produits analogues et leurs mélanges.

27. Composition selon l'une quelconque des revendications précédentes, **caractérisée par le fait qu'**elle présente un pH compris entre 3 et 12.

28. Composition selon l'une quelconque des revendications précédentes, **caractérisée par le fait qu'**elle se présente sous forme de liquides, de crèmes, de gels, ou sous toute autre forme appropriée pour réaliser une teinture des fibres kératiniques, et notamment des cheveux humains.

29. Utilisation des composés de formule (I) ou de leurs sels d'addition avec un acide tels que définis dans l'une quelconque des revendications 1 à 19, à titre de coupleurs dans des compositions pour la teinture d'oxydation des fibres kératiniques et en particulier des fibres kératiniques humaines telles que les cheveux, en association avec au moins un base d'oxydation.

30. Procédé de teinture d'oxydation des fibres kératiniques et en particulier des fibres kératiniques humaines telles que les cheveux, **caractérisé par le fait qu'**on applique sur ces fibres au moins une composition tinctoriale telle que définie à l'une quelconque des revendication 1 à 28, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

31. Procédé selon la revendication 30, **caractérisé par le fait que** l'agent oxydant est choisi parmi le peroxyde d'hydrogène, le peroxyde d'urée, les bromates de métaux alcalins, les persels tels que les perborates et persulfates.

32. Dispositif à plusieurs compartiments, ou "kit" de teinture à plusieurs compartiments, dont un premier compartiment renferme une composition tinctoriale telle que définie à l'une quelconque des revendications 1 à 28 et un second compartiment renferme une composition oxydante.

## Patentansprüche

1. Zusammensetzung zum Färben von Keratinfasern und insbesondere menschlichen Keratinfasern, wie dem Haar, **dadurch gekennzeichnet, daß** sie in einem zum Färben geeigneten Medium enthält:
- als Kuppler mindestens ein Pyrazolo-azol der folgenden Formel (I) oder ein Additionssalz dieser Verbindungen mit einer Säure: 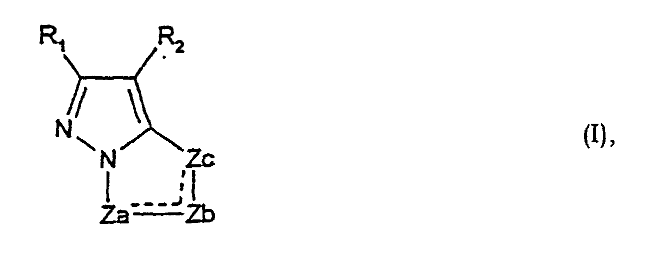 worin bedeuten :
- R₁ Wasserstoff; eine geradkettige oder verzweigte C₁₋₂₀-Alkylgruppe, die gegebenenfalls mit einer oder zwei Gruppen R substituiert ist, die ausgewählt sind unter Halogen, Nitro, Cyano, Hydroxy, Alkoxy, Aryloxy, Amino, Alkylamino, Acylamino, Carbamoyl, Sulfonamido, Sulfamoyl, Imido, Alkylthio, Arylthio, Aryl, Alkoxycarbonyl und Acyl; eine Arylgruppe, die gegebenenfalls mit einer oder zwei der oben definierten Gruppen R substituiert ist; einen Heterocyclus mit 5 oder 6 Gliedern, der mindestens ein Stickstoffatom, Sauerstoffatom oder Schwefelatom aufweist und gegebenenfalls mit einer oder zwei der oben definierten Gruppen R substituiert ist;
wenn R₁ eine Alkylgruppe, eine Arylgruppe oder einen Heterocyclus mit 5 oder 6 Gliedern (die oben definiert sind) bedeutet, kann diese über ein Sauerstoffatom, ein Stickstoffatom oder ein Schwefelatom an das Kohlenstoffatom des Rings gebunden sein (R₁ wird in diesem Fall zu XR₁ mit X = O, NH, S); und R₁ kann auch bedeuten: ein Halogenatom; Acyl; Sulfonyl; Sulfinyl; Phosphonyl; Carbamoyl; Sulfamoyl; Cyano; Siloxy; Amino; Acylamino; Acyloxy; Carbamoyloxy; Sulfonamid; Imid; Ureido; Sulfamoylamino; Alkoxycarbonylamino; Aryloxycarbonylamino; Alkoxycarbonyl; Aryloxycarbonyl; Carboxy;
- R₂ Wasserstoff; ein Halogenatom; Acetylamido; Alkoxy; Aryloxy; Acyloxy; Arylthio; Alkylthio; Heteroarylthio; Heteroaryloxy; Thiocyano; N,N-Diethylthiocarbonylthio; Dodecyloxythiocarbonylthio; Benzolsulfonamido; N-Ethyltoluolsulfonamido; Pentafluorbutanamido; 2,3,4,5,6-Pentafluorbenzamido; p-Cyanophenylureido; N,N-Diethylsulfamoylamino; Pyrazolyl; Imidazolyl; Triazolyl; Tetrazolyl; Benzimidazolyl; 1-Benzyl-5-ethoxy-3-hydantoinyl; 1-Benzyl-3-hydantoinyl; 5,5-Dimethyl-2,4-dioxo-3-oxazolidinyl; 2-Oxy-1,2-dihydro-1-pyridinyl; Alkylamido; Arylamido; Acetylamido; NR^{III}R^{IV,} worin R^{III} und R^{IV}, die identisch oder voneinander verschieden sind, C₁₋₄-Alkyl oder Hydroxyalkyl bedeuten; Carboxy, Alkoxycarboxy;;
- Zₐ, Z_{b} und Z_{c} unabhängig voneinander ein Stickstoffatom oder ein Kohlenstoffatom, das eine Gruppe R₃ oder R₄ trägt, wobei diese Gruppen die für die Gruppe R₁ angegebenen Bedeutungen aufweisen, mit der Maßgabe, daß mindestens eine der Gruppen Zₐ, Z_{b} und Z_{c} kein Kohlenstoffatom bedeutet; die Gruppen R₃ und R₄ können auch gemeinsam einen gegebenenfalls substituierten aromatischen Ring bilden;
und
- mindestens eine Oxidationsbase.

2. Zusammensetzung nach Anspruch 1, **dadurch gekennzeichnet, daß** die Gruppen R₁ der Formel (I) unter Wasserstoff; geradkettigen oder verzweigten C₁₋₄-Alkylgruppen; Phenyl; Phenylgruppen, die mit einem Halogenatom, C₁₋₄-Alkyl, C₁₋₄-Alkoxy, Nitro, Amino, Trifluormethyl oder C₁₋₄-Alkylamino substituiert sind; Benzyl; Benzylgruppen, die mit einem Halogenatom, C₁₋₄-Alkyl, C₁₋₄-Alkoxy, Nitro, Amino, Trifluormethyl oder C₁₋₄-Alkylamino substituiert sind; Heterocyclen, die unter Thiophen, Furan oder Pyridin ausgewählt sind; Trifluormethyl; Gruppen (CH₂)ₚ-X-(CH₂)_{q}-OR', worin p und q, die identisch oder voneinander verschieden sind, ganze Zahlen in Bereich von 1 bis 3 bedeuten, R' H oder Methyl bedeutet und X ein Sauerstoffatom oder eine Gruppe NR" mit R" = Wasserstoff oder Methyl ist; C₁₋₄-Hydroxyalkyl; C₁₋₄-Aminoalkyl; C₁₋₄-Alkylamino; C₁₋₄-Dialkylamino; Arylamino; Alkoxygruppen, die unter Methoxy, Ethoxy oder Phenoxy ausgewählt sind; Halogenatomen, die unter Chlor, Brom oder Fluor ausgewählt sind; Carboxy; C₁₋₄-Alkoxycarbonyl; Phenyloxycarbonyl; Methylthio; Ethylthio; Phenylthio; Methansulfonyl; und Cyano ausgewählt sind.

3. Zusammensetzung nach Anspruch 1 oder 2, **dadurch gekennzeichnet, daß** die Gruppen R₁ der Formel (I) unter Wasserstoff; Alkylgruppen, die unter Methyl, Ethyl, Isopropyl und *t*-Butyl ausgewählt sind; Halogenatomen, die unter Fluor und Chlor ausgewählt sind; Phenyl; Tolyl; 4-Chlorphenyl; 4-Methoxyphenyl; 3-Methoxyphenyl; 2-Methoxyphenyl; Benzyl; Heterocyclen, die unter Pyridyl, Furyl oder Thienyl ausgewählt sind; Trifluormethyl; Hydroxymethyl; Aminomethyl; Methoxy oder Ethoxy; Methylamino oder Ethylamino oder Dimethylamino; Carboxy; Methoxycarbonyl oder Ethoxycarbonyl; und Cyano ausgewählt sind.

4. Zusammensetzung nach Anspruch 3, **dadurch gekennzeichnet, daß** die Gruppen R₁ der Formel (I) unter Wasserstoff; Methyl; Ethyl; Phenyl; Tolyl; 4-Chlorphenyl; 4-Methoxyphenyl; Benzyl; Trifluormethyl; Chlor; Methoxy oder Ethoxy; Carboxy; Methylamino oder Dimethylamino; oder Cyano ausgewählt sind.

5. Zusammensetzung nach einem der Ansprüche 1 bis 4, **dadurch gekennzeichnet, daß** die Gruppen R₂ der Formel (I) unter Wasserstoff; C₁₋₄- Alkoxy; Phenoxy; Phenoxygruppen, die mit einem Halogenatom, C₁₋₄-Alkyl, Carboxy oder Trifluormethyl substituiert sind; Acyloxy; Benzyloxy; C₁₋₄-Alkylthio; Phenylthio; Phenylthiogruppen, die mit einem Halogenatom, C₁₋₄-Alkyl, Carboxy oder Trifluormethyl substituiert sind; C₁₋₄-Alkylamido; Phenylamido; NR^{III}R^{IV}, worin R^{III} und R^{IV}, die identisch oder voneinander verschieden sind, C₁₋₄-Alkyl oder C₁₋ ₄-Hydroxyalkyl bedeuten; Carboxy; oder C₁₋₄-Alkoxycarboxy ausgewählt sind.

6. Zusammensetzung nach Anspruch 5, **dadurch gekennzeichnet, daß** die Gruppen R₁ der Formeln (I) und (II) unter Wasserstoff; Chlor oder Brom; Methoxy oder Ethoxy; Phenoxy; 4-Methylphenyloxy; Acyloxy; Benzyloxy; Methylthio oder Ethylthio; Phenylthio; 4-Methylphenylthio; 2-*t*-Butylphenylthio; Acetamido; Phenylacetamido; Dimethylamino; Diethylamino; Ethylmethylamino; und (β-Hydroxyethyl)methylamino ausgewählt sind.

7. Zusammensetzung nach Anspruch 6, **dadurch gekennzeichnet, daß** die Gruppen R₂ der Formel (I) unter Wasserstoff; Chlor; Ethoxy; Phenoxy; Benzyloxy; Acyloxy; Acetamido und Dimethylamino ausgewählt sind.

8. Zusammensetzung nach einem der Ansprüche 1 bis 7, **dadurch gekennzeichnet, daß** die Gruppen R₃ und R₄ der Formel (I) ausgewählt sind unter:
Wasserstoff; geradkettigen oder verzweigten C₁₋₄-Alkylgruppen; Trifluormethyl; Phenyl; Phenylgruppen, die mit einer oder zwei Gruppen substituiert sind, die unter Halogen, C₁₋₄-Alkyl, C₁₋₄-Alkoxy, Hydroxy, Carboxy, Nitro, C₁₋₄-Alkylthio, Methylendioxy, Amino, Trifluormethyl oder C₁₋₄-Alkylamino ausgewählt sind; Benzyl; Benzylgruppen, die mit einem Halogenatom, Methyl, Isopropyl oder Methoxy substituiert sind; C₁₋₄-Hydroxyalkyl; C₁₋₄-Aminoalkyl; C₁₋₄-Alkylaminoalkyl; einer Alkoxygruppe, die unter Methoxy, Ethoxy oder Phenoxy ausgewählt ist; Methylthio; Ethylthio; Phenylthio; Methansulfonyl; einem gegebenenfalls substituierten aromatischen Ring, der von R₃ und R₄ gebildet wird.

9. Zusammensetzung nach Anspruch 8, **dadurch gekennzeichnet, daß** die Gruppen R₃ und R₄ der Formel (I) ausgewählt sind unter:
Wasserstoff; einer Alkylgruppe, die unter Methyl, Ethyl, Isopropyl, n-Propyl und *t*-Butyl ausgewählt ist; Phenyl; Toluyl; 2-, 3- oder 4-Chlorphenyl; 3- oder 4-Hydroxyphenyl; 3- oder 4-Aminophenyl; 3- oder 4-Methoxyphenyl; 4-Trifluormethylphenyl; Benzyl; Trifluormethyl; Hydroxymethyl; Hydroxyethyl; Hydroxyisopropyl; Aminomethyl; Aminoethyl; Methoxy; Ethoxy; Methylthio; Ethylthio oder einem Phenylring, der von R₃ und R₄ gebildet wird, wie Phenyl.

10. Zusammensetzung nach Anspruch 9, **dadurch gekennzeichnet, daß** die Gruppen R₃ und R₄ der Formel (I) ausgewählt sind unter:
Wasserstoff; Methyl; Ethyl; Isopropyl; Phenyl; 4-Chlorphenyl; 4-Methoxyphenyl; 4-Aminophenyl; Methoxy; Ethoxy; Methylthio; Ethylthio; und einem von R₃ und R₄ gebildeten Phenylring.

11. Zusammensetzung nach einem der Ansprüche 1 bis 10, **dadurch gekennzeichnet, daß** die Verbindungen der Formel (I) ausgewählt sind unter:
(i) Pyrazolo-[1,5-b]-1,2,4-triazolen der Formel: 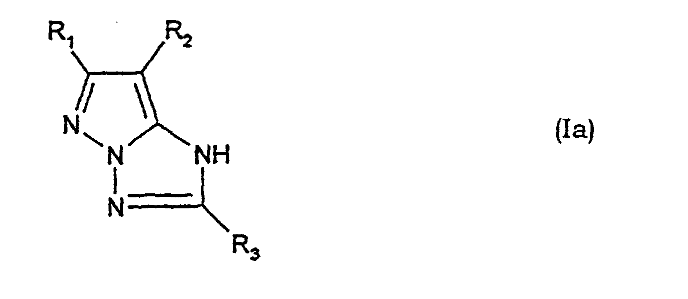
(ii) Pyrazolo-[3,2-c]-1,2,4-triazolen der Formel: 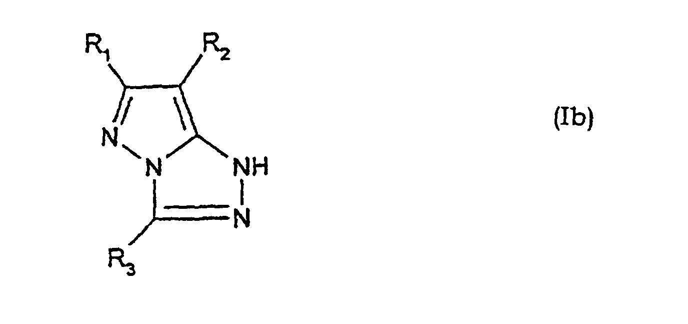
(iii) Pyrazolo-tetrazolen der Formel: 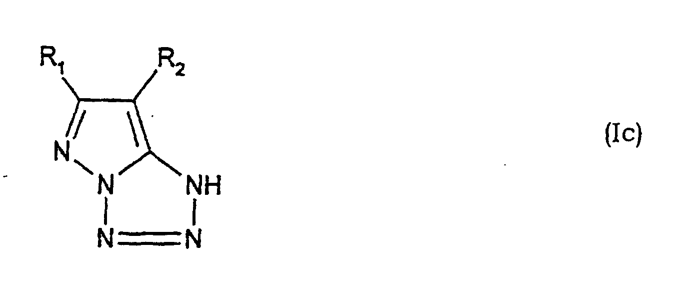
(iv) Pyrazolo-[1,5-a]-imidazolen der Formel: 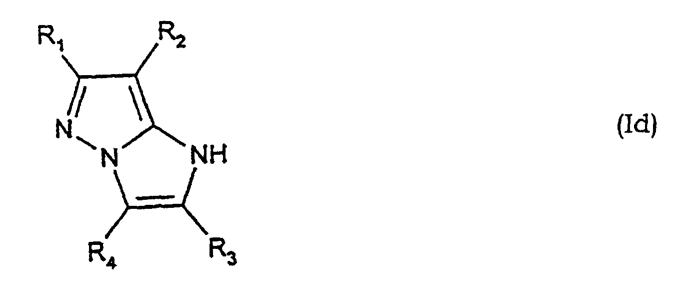
(v) Pyrazolo-[5,1-e]-pyrazolen der Formel: 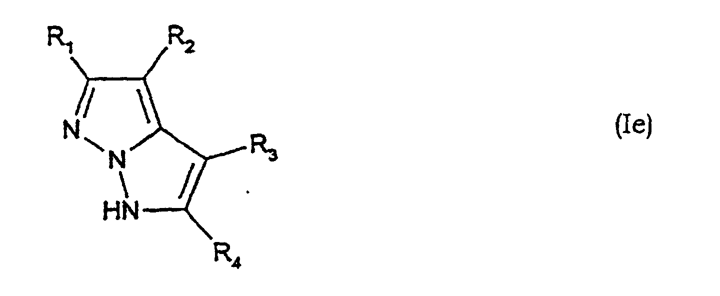
(vi) Pyrazolo-[5,1-e]-1,2,3-triazolen der Formel: 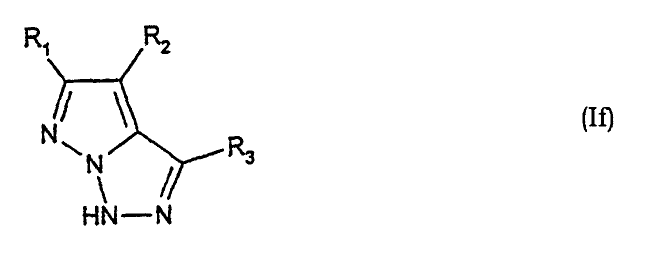
wobei die Gruppen R₁, R₂, R₃ und R₄ die in einem der Ansprüche 1 bis 10 angegebenen Bedeutungen aufweisen,
sowie ihren Additionssalze mit einer Säure.

12. Zusammensetzung nach Anspruch 11, **dadurch gekennzeichnet, daß** die Verbindungen der Formel (Ia) und (Ib) unter den Verbindungen ausgewählt sind, worin bedeuten:
- R₁ Wasserstoff, Methyl, Ethylthio, Amino, Trifluormethyl, Carboxy, Phenyl, Ethoxy oder Cyano;
- R₂ Wasserstoff oder Chlor;
- R₃ Wasserstoff, Methyl, Ethyl, Isopropyl, β-Aminoethyl, β-Hydroxyethyl, Phenyl, Methylthio oder Ethoxy.

13. Zusammensetzung nach Anspruch 11, **dadurch gekennzeichnet, daß** die Verbindungen der Formel (Ic) unter den Verbindungen ausgewählt sind, worin bedeuten:
- R₁ Wasserstoff, Methyl, Trifluormethyl, Carboxy, Phenyl, Ethoxy oder Cyano;
- R₂ Wasserstoff oder Chlor.

14. Zusammensetzung nach Anspruch 11, **dadurch gekennzeichnet, daß** die Verbindungen der Formel (Id) unter den Verbindungen ausgewählt sind, worin bedeuten:
- R₁ Wasserstoff, Methyl, Trifluormethyl, Amino, Carboxy, Phenyl, Ethoxy oder Cyano;
- R₂ Wasserstoff oder Chlor;
- R₃ und R₄: Wasserstoff bzw. Wasserstoff; Wasserstoff und Methyl; Methyl und Wasserstoff; Wasserstoff und Amino; Wasserstoff und Phenyl; oder R₃ und R₄ bilden gemeinsam einen Phenylring.

15. Zusammensetzung nach Anspruch 11, **dadurch gekennzeichnet, daß** die Verbindungen der Formel (Ie) unter den Verbindungen ausgewählt sind, worin bedeuten:
- R₁ Wasserstoff, Methyl, Trifluormethyl, Carboxy, Phenyl, Ethoxy oder Cyano;
- R₂ Wasserstoff oder Chlor;
- R₃ und R₄: Wasserstoff bzw. Methyl; Methyl und Wasserstoff; Methyl und Methyl; oder Wasserstoff und Phenyl.

16. Zusammensetzung nach Anspruch 11, **dadurch gekennzeichnet, daß** die Verbindungen der Formel (If) unter den Verbindungen ausgewählt sind, worin bedeuten:
- R₁ Wasserstoff, Methyl, Trifluormethyl, Carboxy, Phenyl, Ethoxy oder Cyano;
- R₂ Wasserstoff oder Chlor;
- R₃ Wasserstoff oder Methyl.

17. Zusammensetzung nach Anspruch 11, **dadurch gekennzeichnet, daß** die Verbindungen der Formel (I) ausgewählt sind unter:
- 2 -Methyl-pyrazolo-[1,5-b]-1,2,4-triazol,
- 2-Ethyl-pyrazolo-[1,5-b]-1,2,4-triazol,
- 2-Isopropyl-pyrazolo-[1,5-b]-1,2,4-triazol,
- 2-Phenyl-pyrazolo-[1,5-b]-1,2,4-triazol,
- 2,6-Dimethyl-pyrazolo-[1,5-b]-1,2,4-triazol,
- 6-Methyl-2-ethyl-pyrazolo-[1,5-b]-1,2,4-triazol,
- 6-Methyl-2-isopropyl-pyrazolo-[1,5-b]-1,2,4-triazol,
- 6-Methyl-2-phenyl-pyrazolo-[1,5-b]-1,2,4-triazol,
- 6-Carboxy-2-methyl-pyrazolo-[1,5-b]-1,2,4-triazol,
- 6-Carboxy-2-ethyl-pyrazolo-[1,5-b]-1,2,4-triazol,
- 6-Carboxy-2-isopropyl-pyrazolo-[1,5-b]-1,2,4-triazol,
- 6-Carboxy-2-phenyl-pyrazolo-[1,5-b]-1,2,4-triazol,
- 6-Phenyl-2-methyl-pyrazolo-[1,5-b]-1,2,4-triazol,
- 6-Phenyl-2-ethyl-pyrazolo-[1,5-b]-1,2,4-triazol,
- 6-Phenyl-2-isopropyl-pyrazolo-[1,5-b]-1,2,4-triazol,
- 6-Phenyl-2-phenyl-pyrazolo-[1,5-b]-1,2,4-triazol,
- 6-Amino-2-methyl-pyrazolo-[1,5-b]-1,2,4-triazol,
- 6-Amino-2-ethyl-pyrazolo-[1,5-b]-1,2,4-triazol,
- 6-Amino-2-isopropyl-pyrazolo-[1,5-b]-1,2,4-triazol,
- 6-Amino-2-phenyl-pyrazolo-[1,5-b]-1,2,4-triazol,
- 6-Ethylthio-2-methyl-pyrazolo-[1,5-b]-1,2,4-triazol,
- 6-Ethylthio-2-ethyl-pyrazolo-[1,5-b]-1,2,4-triazol,
- 6-Ethylthio-2-isopropyl-pyrazolo-[1,5-b]-1,2,4-triazol,
- 6-Ethylthio-2-phenyl-pyrazolo-[1,5-b]-1,2,4-triazol,
- 6-Ethoxy-2-methyl-pyrazolo-[1,5-b]-1,2,4-triazol,
- 6-Ethoxy-2-ethyl-pyrazolo-[1,5-b]-1,2,4-triazol,
- 6-Ethoxy-2-isopropyl-pyrazolo-[1,5-b]-1,2,4-triazol,
- 6-Ethoxy-2-phenyl-pyrazolo-[1,5-b]-1,2,4-triazol,
- 6-Methyl-2-(2'-aminoethyl)-pyrazolo-[1,5-b]-1,2,4-triazol,
- 6-Carboxy-2-(2'-aminoethyl)-pyrazolo-[1,5-b]-1,2,4-triazol,
- 6-Phenyl-2-(2'-aminoethyl)-pyrazolo-[1,5-b]-1,2,4-triazol,
- 6-Ethylthio-2-(2'-aminoethyl)-pyrazolo-[1,5-b]-1,2,4-triazol,
- 2-(2'-Aminoethyl)-pyrazolo-[1,5-b]-1,2,4-triazol,
- 2-(2'-Hydroxyethyl)-pyrazolo-[1,5-b]-1,2,4-triazol,
- 6-Methyl-2-(2'-hydroxyethyl)-pyrazolo-[1,5-b]-1,2,4-triazol,
- 6-Ethylthio-2-(2'-hydroxyethyl)-pyrazolo-[1,5-b]-1,2,4-triazol,
- 6-Carboxy-2-(2'-hydroxyethyl)-pyrazolo-[1,5-b]-1,2,4-triazol,
- 6-Phenyl-2-(2'-hydroxyethyl)-pyrazolo-[1,5-b]-1,2,4-triazol,
- 7-Chlor-2,6-dimethyl-pyrazolo-[1,5-b]-1,2,4-triazol,
- 7-Brom-2,6-dimethyl-pyrazolo-[1,5-b]-1,2,4-triazol,
- 3-Methyl-pyrazolo-[3,2-c]-1,2,4-triazol,
- 3-Methylsulfinyl-6-phenyl-pyrazolo-[3,2-c]-1,2,4-triazol,
- 3-Ethyl-pyrazolo-[3,2-c]-1,2,4-triazol,
- 3-Isopropyl-pyrazolo-[3,2-c]-1,2,4-triazol,
- 3-Phenyl-pyrazolo-[3,2-c]-1,2,4-triazol,
- 3-(2'-Aminoethyl)-pyrazolo-[3,2-c]-1,2,4-triazol,
- 3-(2'-Hydroxyethyl)-pyrazolo-[3,2-c]-1,2,4-triazol,
- 6-Methyl-3-ethyl-pyrazolo-[3,2-c]-1,2,4-triazol,
- 3,6-Dimethyl-pyrazolo-[3,2-c]-1,2,4-triazol,
- 6-Methyl-3-isopropyl-pyrazolo-[3,2-c]-1,2,4-triazol,
- 6-Methyl-3-phenyl-pyrazolo-[3,2-c]-1,2,4-triazol,
- 6-Methyl-3-(2'-aminoethyl)-pyrazolo-[3,2-c]-1,2,4-triazol,
- 6-Methyl-3-(2'-hydroxyethyl)-pyrazolo-[3,2-c]-1,2,4-triazol,
- 6-Methyl-3-methylthio-pyrazolo-[3,2-c]-1,2,4-triazol,
- 6-Phenyl-3-methyl-pyrazolo-[3,2-c]-1,2,4-triazol,
- 6-Phenyl-3-ethyl-pyrazolo-[3,2-c]-1,2,4-triazol,
- 6-Phenyl-3-isopropyl-pyrazolo-[3,2-c]-1,2,4-triazol,
- 6-Phenyl-3-phenyl-pyrazolo-[3,2-c]-1,2,4-triazol,
- 6-Phenyl-3-(2'-aminoethyl)-pyrazolo-[3,2-c]-1,2,4-triazol,
- 6-Phenyl-3-(2'-hydroxyethyl)-pyrazolo-[3,2-c]-1,2,4-triazol,
- 6-Phenyl-3-methylthio-pyrazolo-[3,2-c]-1,2,4-triazol,
- 6-Ethylthio-3-methyl-pyrazolo-[3,2-c]-1,2,4-triazol,
- 6-Ethylthio-3-ethyl-pyrazolo-[3,2-c]-1,2,4-triazol,
- 6-Ethylthio-3-isopropyl-pyrazolo-[3,2-c]-1,2,4-triazol,
- 6-Ethylthio-3-phenyl-pyrazolo-[3,2-c]-1,2,4-triazol,
- 6-Ethylthio -3-(2'-aminoethyl)-pyrazolo-[3,2-c]-1,2,4-triazol,
- 6-Ethylthio -3-(2'-hydroxyethyl)-pyrazolo-[3,2-c]-1,2,4-triazol,
- 6-Trifluormethyl-3-methylthio-pyrazolo-[3,2-c]-1,2,4-triazol,
- 6-Trifluormethyl-pyrazolo-[3,2-c]-1,2,4-triazol,
- 6-Carboxy-3-methyl-pyrazolo-[3,2-c]-1,2,4-triazol,
- 6-Carboxy-3-ethyl-pyrazolo-[3,2-c]-1,2,4-triazol,
- 6-Carboxy-3-isopropyl-pyrazolo-[3,2-c]-1,2,4-triazol,
- 6-Carboxy-3-phenyl-pyrazolo-[3,2-c]-1,2,4-triazol,
- 6-Carboxy-3-(2'-aminoethyl)-pyrazolo-[3,2-c]-1,2,4-triazol,
- 6-Carboxy-3-(2'-hydroxyethyl)-pyrazolo-[3,2-c]-1,2,4-triazol,
- 7-Chlor-3,6-dimethyl-pyrazolo-[3,2-c]-1,2,4-triazol,
- 7-Methoxycarbonyl-3,6-dimethyl-pyrazolo-[3,2-c]-1,2,4-triazol,
- Pyrazolo-[5,1-e]-tetrazol,
- 6-Methyl-pyrazolo-[5,1-e]-tetrazol,
- 6-Phenyl-pyrazolo-[5,1-e]-tetrazol,
- 6-Carboxy-pyrazolo-[5,1-e]-tetrazol,
- 7-Chlor-6-methyl-pyrazolo-[5,1-e]-tetrazol,
- Pyrazolo-[1,5-a]-imidazol,
- 2-Methyl-pyrazolo-[1,5-a]-imidazol,
- 2-Phenyl-pyrazolo-[1,5-a]-imidazol,
- Pyrazolo-[1,5-al-benzimidazol,
- 6-Methyl-pyrazolo-[1,5-a]-imidazol,
- 2,6-Dimethyl-pyrazolo-[1,5-a]-imidazol,
- 6-Methyl-2-phenyl-pyrazolo-[1,5-a]-imidazol,
- 6-Methyl-pyrazolo-[1,5-a]-benzimidazol,
- 6-Phenyl-pyrazolo-[1,5-a]-imidazol,
- 6-Phenyl-2-methyl-pyrazolo-[1,5-a]-imidazol,
- 2,6-Diphenyl-pyrazolo-[1,5-a]-imidazol,
- 6-Phenyl-pyrazolo-[1,5-a]-benzimidazol,
- 6-Carboxy-pyrazolo-[1,5-a]-imidazol,
- 6-Carboxy-2-methyl-pyrazolo-[1,5-a]-imidazol,
- 6-Carboxy-2-phenyl-pyrazolo-[1,5-a]-imidazol,
- 6-Carboxy-pyrazolo-[1,5-a]-benzimidazol,
- 6-Ethoxy-pyrazolo-[1,5-a]-imidazol,
- 6-Ethoxy-2-methyl-pyrazolo-[1,5-a]-imidazol,
- 6-Ethoxy-2-phenyl-pyrazolo-[1,5-a]-imidazol,
- 6-Trifluormethyl-pyrazolo-[1,5-a]-benzimidazol,
- 6-Amino-pyrazolo-[1,5-a]-imidazol,
- 6-Amino-2-methyl-pyrazolo-[1,5-a]-imidazol,
- 6-Amino-2-phenyl-pyrazolo-[1,5-a]-imidazol,
- 6-Amino-pyrazolo-[1,5-a]-benzimidazol,
- 6-Ethylthio-pyrazolo-[1,5-a]-imidazol,
- 6-Ethylthio-2-methyl-pyrazolo-[1,5-a]-imidazol,
- 6-Ethylthio-2-phenyl-pyrazolo-[1,5-a]-imidazol,
- 7-Chlor-6-methyl-pyrazolo-[1,5-a]-imidazol,
- 7-Chlor-6-methyl-pyrazolo-[1,5-a]-benamidazol,
- 8-Amino-4-methyl-pyrazolo-[5,1-e]-pyrazol,
- 8-Amino-5-chlor-4-methyl-pyrazolo-[5,1-e]-pyrazol,
- 5-Methyl-pyrazolo-[5,1-e]-1,2,3-triazol,
- 5-Methyl-6-chlor-pyrazolo-[5,1-e]-1,2,3-triazol,
- 5-Phenyl-pyrazolo-[5,1-e]-1,2,3-triazol,
- und deren Additionssalze mit einer Säure.

18. Zusammensetzung nach einem der Ansprüche 1 bis 11, **dadurch gekennzeichnet, daß** sie mindestens eine Verbindung der folgenden Formel enthält:

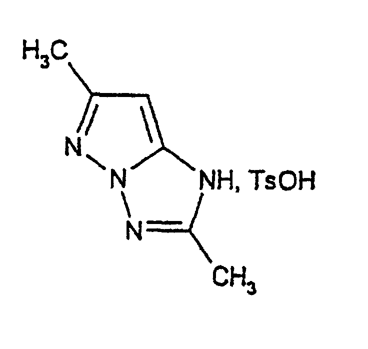

19. Zusammensetzung nach einem der vorhergehenden Ansprüche, **dadurch gekennzeichnet, daß** die Additionssalze der Verbindungen der Formel (I) mit einer Säure unter den Hydrochloriden, Hydrobromiden, Sulfaten, Tartraten, Benzolsulfonaten, Lactaten, Tosylaten und Acetaten ausgewählt sind.

20. Zusammensetzung nach einem der vorhergehenden Ansprüche, **dadurch gekennzeichnet, daß** die Verbindung(en) der Formel (I) 0,0005 bis 12 Gew.-% des Gesamtgewichts der Farbmittelzusammensetzung ausmachen.

21. Zusammensetzung nach Anspruch 20, **dadurch gekennzeichnet, daß** die Verbindung(en) der Formel (I) 0,005 bis 12 Gew.-% des Gesamtgewichts der Farbmittelzusammensetzung ausmachen.

22. Zusammensetzung nach einem der vorhergehenden Ansprüche, **dadurch gekennzeichnet, daß** die Oxidationsbase(n) unter den p-Phenylendiaminen, Bisphenylalkylendiaminen, p-Aminophenolen, o-Aminophenolen, heterocyclischen Basen und deren Additionssalzen mit einer Säure ausgewählt sind.

23. Zusammensetzung nach einem der vorhergehenden Ansprüche, **dadurch gekennzeichnet, daß** die Oxidationsbase(n) etwa 0,0005 bis 12 Gew.-% des Gesamtgewichts der Farbmittelzusammensetzung ausmachen.

24. Zusammensetzung nach Anspruch 23, **dadurch gekennzeichnet, daß** die Oxidationsbase(n) etwa 0,005 bis 6 Gew.-% des Gesamtgewichts der Farbmittelzusammensetzung ausmachen.

25. Zusammensetzung nach einem der vorhergehenden Ansprüche, **dadurch gekennzeichnet, daß** sie ferner einen oder mehrere zusätzliche Kuppler, die von den Verbindungen der Formel (I) verschieden sind, und/oder einen oder mehrere Direktfarbstoffe enthält.

26. Zusammensetzung nach einem der vorhergehenden Ansprüche, **dadurch gekennzeichnet, daß** das zum Färben geeignete Medium (oder der Träger) aus Wasser oder einem Gemisch von Wasser und mindestens einem organischen Lösungsmittel besteht, das unter den niederen C₁₋₄-Alkoholen, Glycerin, Glykolen und Glykolethern, aromatischen Alkoholen, analogen Produkten und deren Gemischen ausgewählt ist.

27. Zusammensetzung nach einem der vorhergehenden Ansprüche, **dadurch gekennzeichnet, daß** sie einen pH-Wert im Bereich von 3 bis 12 aufweist.

28. Zusammensetzung nach einem der vorhergehenden Ansprüche, **dadurch gekennzeichnet, daß** sie als Flüssigkeit, Creme oder Gel oder in beliebigen weiteren Formen, die zur Durchführung einer Färbung von Keratinfasern und insbesondere von menschlichem Haar geeignet sind, vorliegt.

29. Verwendung von Verbindungen der Formel (I) oder von Additionssalzen dieser Verbindungen mit einer Säure nach einem der Ansprüche 1 bis 19 als Kuppler in Zusammensetzungen zum oxidativen Färben von Keratinfasern und insbesondere menschlichen Keratinfasern, wie dem Haar, in Kombination mit mindestens einer Oxidationsbase.

30. Verfahren zum oxidativen Färben von Keratinfasern und insbesondere menschlichen Keratinfasern, wie dem Haar, **dadurch gekennzeichnet, daß** auf die Fasern mindestens eine Farbmittelzusammensetzung nach einem der Ansprüche 1 bis 28 aufgetragen wird und die Farbe bei einem sauren, neutralen oder alkalischen pH-Wert mit einem Oxidationsmittel entwickelt wird, das bei der Anwendung zu der Farbmittelzusammensetzung gegeben wird oder das in einer oxidierenden Zusammensetzung vorliegt, die gleichzeitig oder getrennt davon anschließend aufgetragen wird.

31. Verfahren nach Anspruch 30, **dadurch gekennzeichnet, daß** das Oxidationsmittel unter Wasserstoffperoxid, Harnstoffperoxid, Alkalimetallbromaten und Salzen von Persäuren, wie Perboraten und Persulfaten, ausgewählt ist.

32. Vorrichtung mit mehreren Abteilungen oder "Kit" zum Färben mit mehreren Abteilungen, wobei eine erste Abteilung eine Farbmittelzusammensetzung nach einem der Ansprüche 1 bis 28 und eine zweite Abteilung eine oxidierende Zusammensetzung enthält.

## Claims

1. Composition for dyeing keratinous fibres, and especially human keratinous fibres such as hair, **characterized in that** it comprises, in a medium appropriate for dyeing:
- as coupler, at least one pyrazolo-azole compound of the following formula (I) or one of its addition salts with an acid: 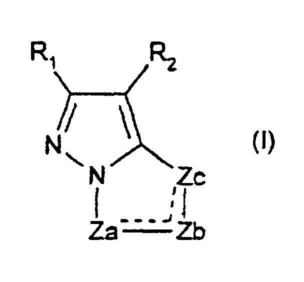 in which
• R₁ is: a hydrogen atom; a linear or branched C₁-C₂₀ alkyl radical which is optionally substituted by one or two radicals R selected from the group consisting of halogen, nitro, cyano, hydroxyl, alkoxy, aryloxy, amino, alkylamino, acylamino, carbamoyl, sulphonamido, sulphamoyl, imido, alkylthio, arylthio, aryl, alkoxycarbonyl and acyl; an aryl radical which is optionally substituted by one or two radicals R as defined above; a 5- or 6-membered heterocycle having at least one nitrogen, oxygen or sulphur atom and being optionally substituted by one or two radicals R as defined above;
when R₁ is an alkyl radical, an aryl radical or a 5- or 6-membered heterocycle (as defined above) it can be attached to the carbon atom of the ring system by way of an oxygen, nitrogen or sulphur atom (in this case R₁ becomes XR₁ where X = O, NH, S);
R₁ may also be a halogen atom; an acyl radical; a sulphonyl radical; a sulphinyl radical; a phosphonyl radical; a carbamoyl radical; a sulphamoyl radical; a cyano radical; a siloxy radical; an amino radical; an acylamino radical; an acyloxy radical; a carbamoyloxy radical; a sulphonamide radical; an imide radical; a ureido radical; a sulphamoylamino radical; an alkoxycarbonylamino radical; an aryloxycarbonyl-amino radical; an alkoxycarbonyl radical; an aryloxycarbonyl radical; or a carboxyl radical;
• R₂ is: a hydrogen atom; a halogen atom; an acetylamido radical; an alkoxy radical; an aryloxy radical; an acyloxy radical; an arylthio radical; an alkythio radical; a heteroarylthio radical; a heteroaryloxy radical; a thiocyano radical; an N,N-diethylthiocarbonylthio radical; a dodecyloxythiocarbonylthio radical; a benzenesulphonamido radical; an N-ethyltoluenesulphonamido radical; a pentafluorobutanamido radical; a 2,3,4,5,6-pentafluorobenzamido radical; a p-cyanophenylureido radical; an N,N-diethylsulphamoylamino radical, a pyrazolyl radical; an imidazolyl radical; a triazolyl radical; a tetrazolyl radical; a benzimidazolyl radical; a 1-benzyl-5-ethoxy-3-hydantoinyl radical; a 1-benzyl-3-hydantoinyl radical; 5,5-dimethyl-2,4-dioxo-3-oxazolidinyl; a 2-oxy-1,2-dihydro-1-pyridinyl radical; an alkylamido; an arylamido; a radical NR^{III}R^{IV} where R^{III} and R^{IV} are identical or different and are a C₁-C₄ alkyl; a hydroxyalkyl; a carboxyl; or an alkoxycarboxyl radical;
Zₐ, Z_{b} and Z_{c} independently of one another are a nitrogen atom, a carbon atom carrying a radical R₃ or R₄ as defined for the radical R₁; with the proviso that at least one of the radicals Zₐ, Z_{b} and Z_{c} is other than a carbon atom; R₃ and R₄ may also together form a substituted or unsubstituted aromatic ring;
• and at least one oxidation base.

2. Composition according to Claim 1, **characterized in that** the radicals R₁ of the formula (I) are selected from the group consisting of:
a hydrogen atom; a linear or branched C₁-C₄ alkyl; a phenyl; a phenyl substituted by a halogen atom, a C₁-C₄ alkyl, a C₁-C₄ alkoxy, a nitro group, an amino group, a trifluoromethyl group or C₁-C₄ alkylamino group; a benzyl radical; a benzyl radical substituted by a halogen atom, a C₁-C₄ alkyl, a C₁-C₄ alkoxy, a nitro group, an amino group or a trifluoromethyl group; a C₁-C₄ alkylamino; a heterocycle selected from thiophene, furan and pyridine; a trifluoromethyl radical; a radical (CH₂)ₚ-X-(CH₂)_{q}-OR' where p and q are identical or different integers from 1 to 3, R' is H or methyl and X is an oxygen atom or a group NR" where R" is hydrogen or methyl; a C₁-C₄ hydroxyalkyl; a C₁-C₄ aminoalkyl; a C₁-C₄ alkylamino; a C₁-C₄ dialkylamino; an arylamino; an alkoxy radical selected from methoxy, ethoxy and phenoxy; a halogen selected from fluorine, chlorine and bromine; a carboxyl group; a C₁-C₄ alkoxycarbonyl; a phenyloxycarbonyl; methylthio; ethylthio; phenylthio; methanesulphonyl; and cyano.

3. Composition according to Claim 1 or 2, **characterized in that** the radicals R₁ of the formula (I) are selected from the group consisting of:
hydrogen; an alkyl selected from methyl, ethyl, isopropyl and tert-butyl; a halogen selected from fluorine and chlorine; phenyl; tolyl; 4-chlorophenyl; 4-methoxyphenyl; 3-methoxyphenyl; 2-methoxyphenyl; benzyl; a heterocycle selected from pyridyl, furyl and thienyl; trifluoromethyl; hydroxymethyl; aminomethyl; methoxy or ethoxy; methylamino or ethylamino or dimethylamino; carboxyl; methoxycarbonyl or ethoxycarbonyl; and cyano.

4. Composition according to Claim 3, **characterized in that** the radicals R₁ of the formula (I) are selected from the group consisting of:
hydrogen; methyl; ethyl; phenyl; tolyl; 4-chlorophenyl; 4-methoxyphenyl; benzyl; trifluoromethyl; chloro; a methoxy or ethoxy radical; a carboxyl radical; a methylamino or dimethylamino radical; and cyano.

5. Composition according to any one of Claims 1 to 4, **characterized in that** the radicals R₂ of the formula (I) are selected from the group consisting of:
a hydrogen atom; a C₁-C₄ alkoxy; phenoxy; phenoxy substituted by a halogen atom, a C₁-C₄ alkyl, a carboxyl or a trifluoromethyl group; an acyloxy radical; benzyloxy; C₁-C₄ alkylthio; phenylthio; phenylthio substituted by a halogen atom, a C₁-C₄ alkyl, a carboxyl or a trifluoromethyl group; a C₁-C₄ alkylamido; phenylamido; a radical NR^{III}R^{IV} where R^{III} and R^{IV} are identical or different and are a C₁-C₄ alkyl or a C₁-C₄ hydroxyalkyl; a carboxyl; and a C₁-C₄ alkoxycarboxyl radical.

6. Composition according to Claim 5, **characterized in that** the radicals R₂ of the formula (I) are selected from the group consisting of:
hydrogen; chlorine or bromine; methoxy or ethoxy; phenyloxy; 4-methylphenyloxy; acyloxy; benzyloxy; methylthio or ethylthio; phenylthio; 4-methylphenylthio; 2-tert-butylphenylthio; acetamido; phenylacetamido; dimethylamino; diethylamino; ethylmethylamino; and (β-hydroxyethyl)methylamino.

7. Composition according to Claim 6, **characterized in that** the radicals R₂ of the formula (I) are selected from the group consisting of:
hydrogen; chlorine; ethoxy; phenoxy; benzyloxy; acyloxy; acetamido; and dimethylamino.

8. Composition according to any one of Claims 1 to 7, **characterized in that** the radicals R₃ and R₄ of the formula (I) are selected from the group consisting of:
a hydrogen atom; a linear or branched C₁-C₄ alkyl; a trifluoromethyl; a phenyl; a phenyl substituted by one or two groups selected from a halogen, a C₁-C₄ alkyl, a C₁-C₄ alkoxy, a hydroxyl, a carboxyl, a nitro group, a C₁-C₄ alkylthio, a methylenedioxy group, an amino group, a trifluoromethyl group or a C₁-C₄ alkylamino; a benzyl radical; a benzyl radical substituted by a halogen atom, a methyl or isopropyl, or methoxy; a C₁-C₄ hydroxyalkyl; a C₁-C₄ aminoalkyl; a C₁-C₄ alkylaminoalkyl; an alkoxy radical selected from methoxy, ethoxy and phenoxy; methylthio; ethylthio; phenylthio; methanesulphonyl; and a substituted or unsubstituted aromatic ring formed by R₃ and R₄.

9. Composition according to Claim 8, **characterized in that** the radicals R₃ and R₄ of the formula (I) are selected from the group consisting of:
hydrogen; an alkyl selected from methyl, ethyl, isopropyl, n-propyl and tert-butyl; phenyl; tolyl; 2-, 3- or 4-chlorophenyl; 3- or 4-hydroxyphenyl; 3- or 4-aminophenyl; 3- or 4-methoxyphenyl; 4-trifluoromethylphenyl; benzyl; trifluoromethyl; hydroxymethyl; hydroxyethyl; hydroxyisopropyl; aminomethyl or aminoethyl; methoxy or ethoxy; methylthio or ethylthio; and a phenyl ring formed by R₃ and R₄, such as phenyl.

10. Composition according to Claim 9, **characterized in that** the radicals R₃ and R₄ of the formula (I) are selected from the group consisting of:
hydrogen; methyl; ethyl; isopropyl; phenyl; 4-chlorophenyl; 4-methoxyphenyl; 4-aminophenyl; methoxy or ethoxy; methylthio or ethylthio; and a phenyl ring formed by R₃ and R₄.

11. Composition according to any one of Claims 1 to 10, **characterized in that** the compounds of formula (I) are selected from the group consisting of:
i) the pyrazolo[1,5-b]-1,2,4-triazoles of formula: 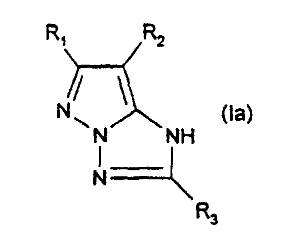
ii) the pyrazolo[3,2-c]-1,2,4-triazoles of formula: 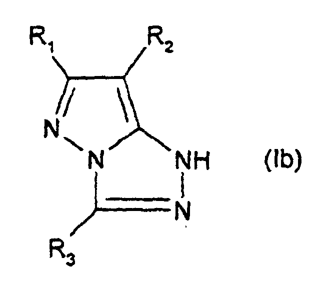
iii) the pyrazolotetrazoles of formula: 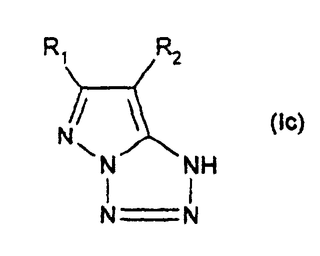
iv) the pyrazolo[1,5-a]imidazoles of formula: 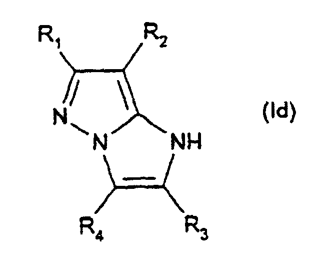
v) the pyrazolo[5,1-e]pyrazoles of formula: 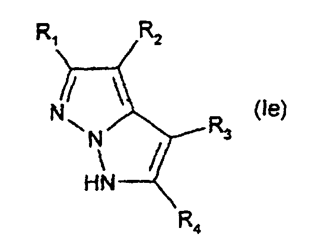
vi) the pyrazolo[5,1-e]-1,2,3-triazoles of formula: 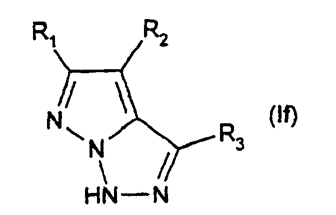
in which R₁, R₂, R₃ and R₄ are as defined in any one of Claims 1 to 10; and their addition salts with an acid.

12. Composition according to Claim 11, **characterized in that** the compounds of formula (Ia) or (Ib) are selected from those for which:
- R₁ is hydrogen, methyl, ethylthio, amino, trifluoromethyl, carboxyl, phenyl, ethoxy or cyano;
- R₂ is hydrogen or chlorine; and
- R₃ is hydrogen, methyl, ethyl, isopropyl, β-aminoethyl, β-hydroxyethyl, phenyl, methylthio or ethoxy.

13. Composition according to Claim 11, **characterized in that** the compounds of formula (Ic) are selected from those for which:
- R₁ is hydrogen, methyl, trifluoromethyl, carboxyl, phenyl, ethoxy or cyano; and
- R₂ is hydrogen or chlorine.

14. Composition according to Claim 11, **characterized in that** the compounds of formula (Id) are selected from those for which:
- R₁ is hydrogen, methyl, trifluoromethyl, amino, carboxyl, phenyl, ethoxy or cyano;
- R₂ is hydrogen or chlorine; and
- R₃ and R₄ are respectively hydrogen and hydrogen, hydrogen and methyl, methyl and hydrogen, hydrogen and amino, or hydrogen and phenyl; or together form a phenyl ring.

15. Composition according to Claim 11, **characterized in that** the compounds of formula (Ie) are selected from those for which:
- R₁ is hydrogen, methyl, trifluoromethyl, carboxyl, phenyl, ethoxy or cyano;
- R₂ is hydrogen or chlorine; and
- R₃ and R₄ are respectively hydrogen and methyl, methyl and hydrogen, methyl and methyl or hydrogen and phenyl.

16. Composition according to Claim 11, **characterized in that** the compounds of formula (If) are selected from those for which:
- R₁ is hydrogen, methyl, trifluoromethyl, carboxyl, phenyl, ethoxy or cyano;
- R₂ is hydrogen or chlorine; and
- R₃ is hydrogen or methyl.

17. Composition according to Claim 11, **characterized in that** the compounds of formula (I) are selected from:
- 2-methylpyrazolo[1,5-b]-1,2,4-triazole,
- 2-ethylpyrazolo[1,5-b]-1,2,4-triazole,
- 2-isopropylpyrazolo[1,5-b]-1,2,4-triazole,
- 2-phenylpyrazolo[1,5-b]-1,2,4-triazole,
- 2,6-dimethylpyrazolo[1,5-b]-1,2,4-triazole,
- 6-methyl-2-ethylpyrazolo[1,5-b] -1,2,4-triazole,
- 6-methyl-2-isopropylpyrazolo[1,5-b]-1,2,4-triazole,
- 6-methyl-2-phenylpyrazolo [1,5-b]-1,2,4-triazole,
- 6-carboxy-2-methylpyrazolo[1,5-b]-1,2,4-triazole,
- 6-carboxy-2-ethylpyrazolo[1,5-b]-1,2,4-triazole,
- 6-carboxy-2-isopropylpyrazolo[1,5-b]-1,2,4-triazole,
- 6-carboxy-2-phenylpyrazolo[1,5-b]-1,2,4-triazole,
- 6-phenyl-2-methylpyrazolo[1,5-b]-1,2,4-triazole,
- 6-phenyl-2-ethylpyrazolo[1,5-b]-1,2,4-triazole,
- 6-phenyl-2-isopropylpyrazolo[1,5-b]-1,2,4-triazole,
- 6-phenyl-2-phenylpyrazolo[1,5-b]-1,2,4-triazole,
- 6-amino-2-methylpyrazolo[1,5-b]-1,2,4-triazole,
- 6-amino-2-ethylpyrazolo[1,5-b]-1,2,4-triazole,
- 6-amino-2-isopropylpyrazolo[1,5-b]-1,2,4-triazole,
- 6-amino-2-phenylpyrazolo[1,5-b]-1,2,4-triazole,
- 6-ethylthio-2-methylpyrazolo[1,5-b]-1,2,4-triazole,
- 6-ethylthio-2-ethylpyrazolo[1,5-b]-1,2,4-triazole,
- 6-ethylthio-2-isopropylpyrazolo[1,5-b]-1,2,4-triazole,
- 6-ethylthio-2-phenylpyrazolo[1,5-b]-1,2,4-triazole,
- 6-ethoxy-2-methylpyrazolo[1,5-b]-1,2,4-triazole,
- 6-ethoxy-2-ethylpyrazolo[1,5-b]-1,2,4-triazole,
- 6-ethoxy-2-isopropylpyrazolo[1,5-b]-1,2,4-triazole,
- 6-ethoxy-2-phenylpyrazolo[1,5-b]-1,2,4-triazole,
- 6-methyl-2-(2'-aminoethyl)pyrazolo[1,5-b]-1,2,4-triazole,
- 6-carboxy-2-(2'-aminoethyl)pyrazolo[1,5-b]-1,2,4-triazole,
- 6-phenyl-2-(2'-aminoethyl)pyrazolo[1,5-b]-1,2,4-triazole,
- 6-ethylthio-2-(2'-aminoethyl)pyrazolo[1,5-b]-1,2,4-triazole,
- 2-(2'-aminoethyl)pyrazolo[1,5-b]-1,2,4-triazole,
- 2-(2'-hydroxyethyl)pyrazolo[1,5-b]-1,2,4-triazole,
- 6-methyl-2-(2'-hydroxyethyl)pyrazolo[1,5-b]-1,2,4-triazole,
- 6-ethylthio-2-(2'-hydroxyethyl)pyrazolo[1,5-b]-1,2,4-triazole,
- 6-carboxy-2-(2'-hydroxyethyl)pyrazolo[1,5-b]-1,2,4-triazole,
- 6-phenyl-2-(2'-hydroxyethyl)pyrazolo[1,5-b]-1,2,4-triazole,
- 7-chloro-2,6-dimethylpyrazolo[1,5-b]-1,2,4-triazole,
- 7-bromo-2,6-dimethylpyrazolo [1,5-b]-1,2,4-triazole,
- 3-methylpyrazolo[3,2-c]-1,2,4-triazole,
- 3-methylsulphinyl-6-phenylpyrazolo[3,2-c]-1,2,4-triazole,
- 3-ethylpyrazolo[3,2-c]-1,2,4-triazole,
- 3-isopropylpyrazolo[3,2-c]-1,2,4-triazole,
- 3-phenylpyrazolo[3,2-c]-1,2,4-triazole,
- 3-(2'-aminoethyl)pyrazolo[3,2-c]-1,2,4-triazole,
- 3-(2'-hydroxyethyl)pyrazolo[3,2-c]-1,2,4-triazole,
- 6-methyl-3-ethylpyrazolo[3,2-c]-1,2,4-triazole,
- 3,6-dimethylpyrazolo[3,2-c]-1,2,4-triazole,
- 6-methyl-3-isopropylpyrazolo[3,2-c]-1,2,4-triazole,
- 6-methyl-3-phenylpyrazolo[3,2-c]-1,2,4-triazole,
- 6-methyl-3-(2'-aminoethyl)pyrazolo[3,2-c]-1,2,4-triazole,
- 6-methyl-3-(2'-hydroxyethyl)pyrazolo[3,2-c]-1,2,4-triazole,
- 6-methyl-3-methylthiopyrazolo[3,2-c]-1,2,4-triazole,
- 6-phenyl-3-methylpyrazolo[3,2-c]-1,2,4-triazole,
- 6-phenyl-3-ethylpyrazolo[3,2-c]-1,2,4-triazole,
- 6-phenyl-3-isopropylpyrazolo[3,2-c]-1,2,4-triazole,
- 6-phenyl-3-phenylpyrazolo[3,2-c]-1,2,4-triazole,
- 6-phenyl-3-(2'-aminoethyl)pyrazolo[3,2-c]-1,2,4-triazole,
- 6-phenyl-3-(2'-hydroxyethyl)pyrazolo[3,2-c]-1,2,4-triazole,
- 6-phenyl-3-methylthiopyrazolo[3,2-c]-1,2,4-triazole,
- 6-ethylthio-3-methylpyrazolo[3,2-c]-1,2,4-triazole,
- 6-ethylthio-3-ethylpyrazolo[3,2-c]-1,2,4-triazole,
- 6-ethylthio-3-isopropylpyrazolo[3,2-c]-1,2,4-triazole,
- 6-ethylthio-3-phenylpyrazolo[3,2-c]-1,2,4-triazole,
- 6-ethylthio-3- (2'-aminoethyl)pyrazolo[3,2-c]--1,2,4-triazole,
- 6-ethylthio-3-(2'-hydroxyethyl)pyrazolo[3,2-c]-1,2,4-triazole,
- 6-trifluoromethyl-3-methylthiopyrazolo[3,2-c]-1,2,4-triazole,
- 6-trifluoromethylpyrazolo[3,2-c]-1,2,4-triazole,
- 6-carboxy-3-methylpyrazolo[3,2-c]-1,2,4-triazole,
- 6-carboxy-3-ethylpyrazolo[3,2-c]-1,2,4-triazole,
- 6-carboxy-3-isopropylpyrazolo[3,2-c]-1,2,4-triazole,
- 6-carboxy-3-phenylpyrazolo[3,2-c]-1,2,4-triazole,
- 6-carboxy-3-(2'-aminoethyl)pyrazolo[3,2-c]-1,2,4-triazole,
- 6-carboxy-3-(2'-hydroxyethyl)pyrazolo[3,2-c]-1,2,4-triazole,
- 7-chloro-3,6-dimethylpyrazolo[3,2-c]-1,2,4-triazole,
- 7-methoxycarbonyl-3,6-dimethylpyrazolo[3,2-c]-1,2,4-triazole,
- pyrazolo[5,1-e]tetrazole,
- 6-methylpyrazolo[5,1-e]tetrazole,
- 6-phenylpyrazolo[5,1-e]tetrazole,
- 6-carboxypyrazolo[5,1-e]tetrazole,
- 7-chloro-6-methylpyrazolo[5,1-e]tetrazole,
- pyrazolo[1,5-a]imidazole,
- 2-methylpyrazolo[1,5-a]imidazole,
- 2-phenylpyrazolo[1,5-a]imidazole,
- pyrazolo[1,5-a]benzimidazole,
- 6-methylpyrazolo[1,5-a]imidazole,
- 2,6-dimethylpyrazolo[1,5-a]imidazole,
- 6-methyl-2-phenylpyrazolo[1,5-a]imidazole,
- 6-methylpyrazolo[1,5-a]benzimidazole,
- 6-phenylpyrazolo[1,5-a]imidazole,
- 6-phenyl-2-methylpyrazolo[1,5-a]imidazole,
- 2,6-diphenylpyrazolo[1,5-a]imidazole,
- 6-phenylpyrazolo[1,5-a]benzimidazole,
- 6-carboxypyrazolo[1,5-a]imidazole,
- 6-carboxy-2-methylpyrazolo[1,5-a]imidazole,
- 6-carboxy-2-phenylpyrazolo[1,5-a]imidazole,
- 6-carboxypyrazolo[1,5-a]benzimidazole,
- 6-ethoxypyrazolo[1,5-a]imidazole,
- 6-ethoxy-2-methylpyrazolo[1,5-a]imidazole,
- 6-ethoxy-2-phenylpyrazolo[1,5-a]imidazole,
- 6-trifluoromethylpyrazolo[1,5-a]benzimidazole,
- 6-aminopyrazolo[1,5-a]imidazole,
- 6-amino-2-methylpyrazolo[1,5-a]imidazole,
- 6-amino-2-phenylpyrazolo[1,5-a]imidazole,
- 6-aminopyrazolo[1,5-a]benzimidazole,
- 6-ethylthiopyrazolo[1,5-a]imidazole,
- 6-ethylthio-2-methylpyrazolo[1,5-a]imidazole,
- 6-ethylthio-2-phenylpyrazolo[1,5-a]imidazole,
- 7-chloro-6-methylpyrazolo[1,5-a]imidazole,
- 7-chloro-6-methylpyrazolo[1,5-a]benzimidazole,
- 8-amino-4-methylpyrazolo[5,1-e]pyrazole,
- 8-amino-5-chloro-4-methylpyrazolo[5,1-e]pyrazole,
- 5-methylpyrazolo[5,1-e]-1,2,3-triazole,
- 5-methyl-6-chloropyrazolo[5,1-e]-1,2,3-triazole,
- 5-phenylpyrazolo[5,1-e]-1,2,3-triazole,
and their addition salts with an acid.

18. Composition according to any one of Claims 1 to 11, **characterized in that** it comprises at least one compound of formula:

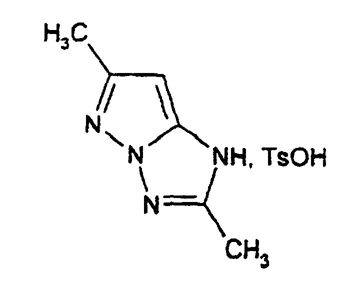

19. Composition according to any one of the preceding claims, **characterized in that** the acid addition salts of the compounds of formula (I) are selected from hydrochlorides, hydrobromides, sulphates, tartrates, benzenesulphonates, lactates, tosylates and acetates.

20. Composition according to any one of the preceding claims, **characterized in that** the compound or compounds of formula (I) represent from 0.0005 to 12% by weight of the total weight of the dyeing composition.

21. Composition according to Claim 20, **characterized in that** the compound or compounds of formula (I) represent from 0.005 to 6% by weight of the total weight of the dyeing composition.

22. Composition according to any one of the preceding claims, **characterized in that** the oxidation base or bases are selected from para-phenylenediamines, bisphenylalkylenediamines, para-aminophenols, ortho-aminophenols, heterocyclic bases, and their addition salts with an acid.

23. Composition according to any one of the preceding claims, **characterized in that** the oxidation base or bases represent from 0.0005 to 12% by weight, approximately, of the total weight of the dyeing composition.

24. Composition according to Claim 23, **characterized in that** the oxidation base or bases represent from 0.005 to 6% by weight, approximately, of the total weight of the dyeing composition.

25. Composition according to any one of the preceding claims, **characterized in that** it also comprises one or more additional couplers other than the compounds of formula (I) and/or one or more direct dyes.

26. Composition according to any one of the preceding claims, **characterized in that** the medium appropriate for dyeing (or vehicle) consists of water or of a mixture of water and at least one organic solvent selected from lower C₁-C₄ alcohols, glycerol, glycols and glycol ethers, aromatic alcohols, similar products, and mixtures thereof.

27. Composition according to any one of the preceding claims, **characterized in that** it has a pH of between 3 and 12.

28. Composition according to any one of the preceding claims, **characterized in that** it is in the form of liquids, creams, gels or any other form which is appropriate for producing a dye for keratinous fibres and especially human hair.

29. Use of the compounds of formula (I) or of their addition salts with an acid, as defined in any one of Claims 1 to 19, as couplers in compositions for the oxidation dyeing of keratinous fibres, and especially human keratinous fibres such as hair, in combination with at least one oxidation base.

30. Method of oxidation-dyeing keratinous fibres, and especially human keratinous fibres such as hair, **characterized in that** at least one dyeing composition as defined in any one of Claims 1 to 28 is applied to these fibres, the colour being developed at an acidic, neutral or alkaline pH with the aid of an oxidizing agent which is added right at the time when the dyeing composition is employed or which is present in an oxidizing composition which is applied simultaneously or sequentially and separately.

31. Method according to Claim 30, **characterized in that** the oxidizing agent is selected from hydrogen peroxide, urea peroxide, alkali metal bromates, and persalts, such as perborates and persulphates.

32. Multi-compartment device, or multi-compartment dyeing kit, a first compartment of which contains a dyeing composition as defined in any one of Claims 1 to 28 and a second compartment of which contains an oxidizing composition.
